# EUROPEAN PATENT APPLICATION

(11) **EP 4 524 228 A1**
(43) Date of publication of application: **19.03.2025**
(21) Application number: 23811882.2
(22) Date of filing: 25.05.2023
(51) Int. Cl.: C12M 1/00, C12M 1/34, C12M 1/42, C12Q 1/04, C12Q 1/68

(54) **METHOD AND DEVICE FOR DETECTING MICROORGANISMS**

(30) Priority: 26.05.2022 US 202263345969 P
(71) Applicant: Kikkoman Corporation, Noda-shi, Chiba 278-8601 (JP)
(72) Inventor: MAEDA, Yasukazu, Noda-shi, Chiba 278-8601 (JP); ISHIKAWA, Yuki, Noda-shi, Chiba 278-8601 (JP); ICHIYANAGI, Atsushi, Noda-shi, Chiba 278-8601 (JP)
(74) Representative: Mewburn Ellis LLP
(86) International application number: PCT/JP2023/019510
(87) International publication number: WO 2023/229010

(57) **Abstract**

A microfluidic device 1 according to the present invention for detecting microorganisms in a sample includes a lysis section 2 for dissolving microorganisms in the sample; and a separation section 3 for separating out a nucleic acid from a lysate of microorganisms formed in the lysis section 2 communicating with the lysis section 2, in which the lysis section 2 includes a channel for holding a bacteriolytic agent for lysing microorganisms, and the separation section 3 includes a means capable of separating out a nucleic acid contained in the lysate from other substances.

## Description

### Technical Field

The present invention relates to a detection method and a detection device for detecting microorganisms.

### Background Art

PCR, ATP assays, immunological detection methods, and the like have been mainly used to measure risk microorganisms in foods. However, although the PCR has good specificity and detection sensitivity, the operation is complicated and a dedicated device is required, and the ATP assay methods cannot specifically detect microorganisms, and the immunological detection method has sensitivity issues, and none of the methods simultaneously satisfied the three requirements of specificity, sensitivity, and simplicity of operation.

The microchannel is a technology that can be utilized in a field of cell separation and cell chemical treatment. For example, in Patent Literature 1, microorganisms are detected using a paper microfluidic chip, but only five types of microorganisms are detected at the genera level, and the microorganisms cannot be discriminated at the microbial species level. In addition, a highly concentrated bacterial sample is required, and it is not satisfactory in terms of specificity and sensitivity.

In addition, in Patent Literature 2, by injecting a prepared sample into a microchannel, microorganisms are confined in minute capsules, one cell at a time, and then recovered and lysed to decode the genome sequence. In this method, sample preparation and operation after microorganism recovery are relatively complicated.

On the other hand, a nanopore is a technology capable of detecting a minute object. In Non Patent Literature 1, nucleic acid detection is performed using holes formed on a SiN base using a TEM, which are called solid state nanopores.

### Citation List

### Patent Literature

Patent Literature 1: US 2020/0298233 A1
Patent Literature 2: US 2021/0246495 A1
Patent Literature 3: Japanese Unexamined Patent Publication No. 2017-530695

### Non Patent Literature

Non Patent Literature 1: YANG, Wayne, et al., Detection of CRISPR-dCas9 on DNA with solid-state nanopores, Nano letters, 2018, 18.10: 6469-6474.

### Summary of Invention

### Technical Problem

As described above, conventionally, techniques such as separation by a microchannel and detection by nanopores are known, but hitherto, no technique has been known that can connect a microchannel and a nanopore to achieve separation and detection of microorganisms at a satisfactory level in terms of specificity, sensitivity, and simplicity of operation.

Therefore, an object of the present invention is to provide a simple detection method and a detection device for specifically and sensitively detecting microorganisms.

### Solution to Problem

The present inventors have intensively studied to provide a simple detection method and a detection device for specifically and sensitively detecting microorganisms, as a result, have found a microfluidic device including a lysis section for dissolving microorganisms and a separation section for separating out a nucleic acid from a lysate of microorganisms formed in the lysis section communicating with the lysis section, and have completed the present invention.

The present invention relates to, for example, the following [1] to [9].
[1] A microfluidic device for detecting microorganisms in a sample, including:
   a lysis section for dissolving microorganisms in the sample; and
   a separation section for separating out a nucleic acid from a lysate of microorganisms formed in the lysis section communicating with the lysis section,
   in which the lysis section includes a channel for holding a bacteriolytic agent for lysing microorganisms, and
   the separation section includes a separation means capable of separating out a nucleic acid contained in the lysate from other substances.
[2] The device according to [1], in which the bacteriolytic agent contains a surfactant.
[3] The device according to [1] or [2], in which the separation means of the separation section is a separation means using a molecular size, a separation means using centrifugation, or a separation means using hydraulic filtration.
[4] The device according to any one of [1] to [3], further including:
   a complex forming section for forming a protein-nucleic acid complex from the nucleic acid separated in the separation section communicating with the separation section; and
   a detection section including a means for detecting the complex communicating with the complex forming section.
[5] The device according to [4], in which the complex forming section includes a channel holding a specific nucleic acid derived from the microorganisms and a substance capable of forming a protein-nucleic acid complex.
[6] The device according to [5], in which the substance is CRISPR-dCas9 to which sgRNA specific to the specific nucleic acid is bound, or a protein marker that specifically binds to the specific nucleic acid.
[7] The device according to any one of [4] to [6], in which the detection section includes a means for electrochemically or optically detecting the complex.
[8] The device according to any one of [4] to [6], in which the detection section detects the complex using a nanopore.
[9] The device according to any one of [1] to [3], further including:
   a detection section including a means for detecting the nucleic acid separated in the separation section communicating with the separation section.
[10] The device according to [9], in which the detection section detects the nucleic acid by real-time PCR or an immunological method.
[11] A method for detecting microorganisms in a sample using the device according to any one of [1] to [8].
[12] The method according to [11], including:
   a lysis step of lysing microorganisms in a sample in the lysis section; and
   a separation step of separating out a nucleic acid from a lysate of microorganisms obtained in the lysis section, in the separation section.
[13] The method according to [12], further including:
   a complex forming step of forming a protein-nucleic acid complex from the nucleic acid separated in the separation section, in the complex forming section.
[14] The method according to [13], further including:
   a detection step of detecting the protein-nucleic acid complex formed in the complex forming section, in the detection section.
[15] A method for detecting microorganisms in a sample using the device according to any one of [1] to [3], [9], and [10].
[16] The method according to [15], including:
   a lysis step of lysing microorganisms in a sample in the lysis section;
   a separation step of separating out a nucleic acid from a lysate of microorganisms in the separation section; and
   a detection step of detecting the nucleic acid separated in the separation section, in the detection section.

### Advantageous Effects of Invention

According to the detection device of the present invention and the detection method using the device, it is possible to easily detect microorganisms specifically and sensitively by a series of operations. The present invention is particularly conveniently used for detecting food poisoning microorganisms, and therefore can be widely and suitably used in various fields such as food production, distribution, and retail, medical, dental, and pharmaceutical fields, and for various environmental measurements.

### Brief Description of Drawings

FIG. 1 is a schematic diagram of (a) Channel 6 and (b) Channel 1 according to an embodiment in Example 1.
FIG. 2 is a microscopic image showing results of verifying a lytic effect using four kinds of bacteriolytic agents in Example 1.
FIG. 3 is a graph showing a lysis rate of Bacillus cereus using SDS in Example 1.
FIG. 4 is a graph showing a lysis rate of Bacillus cereus using SDS and Proteinase K in Example 1.
FIG. 5 is an electrophoretic photograph showing results of verifying an influence of SDS on the formation of a complex in Example 1.
FIG. 6 is a graph showing evaluation results of current inhibition by a formed nucleic acid-protein complex in Example 1.
FIG. 7 shows a device 1 according to an embodiment.
FIG. 8 shows a mechanism of microorganism detection by a device according to an embodiment.
FIG. 9 shows a mechanism of detection of a complex by nanopores.
FIG. 10 shows various lysis section channel structures in Example 2.
FIG. 11 is a graph showing the degree of lysis (viability rate) by various lysis section channel structures in Example 2.
FIG. 12 shows various bacteriolytic agents in Example 3.
FIG. 13 is a graph showing the degree of lysis (viability rate) of various bacteriolytic agents in Example 3.
FIG. 14 is a graph showing the degree of lysis (nucleic acid recovery rate) by BugBuster and sulfobetaine in Example 3.
FIG. 15 is a graph showing the degree of lysis (nucleic acid recovery rate) of various bacteriolytic agents in Example 3.
FIG. 16 is a graph showing results of examining combinations of lysis section channel structures and bacteriolytic agents in Example 4.
FIG. 17 is a graph showing the degree of lysis (nucleic acid recovery rate) with respect to other food poisoning bacteria in Example 5.
FIG. 18 is a conceptual diagram of complex formation using a FRET evaluation system in Example 6.
FIG. 19 shows various channel structures of a complex forming section in Example 7.
FIG. 20 is a graph showing a complex formation rate obtained using various channel structures in Example 7.
FIG. 21 shows three kinds of pretreatment integrated channels in Example 8.
FIG. 22 shows a detailed structure of a pretreatment integrated channel ver. 2-1 shown in FIG. 20(B) in Example 8.
FIG. 23 shows a detailed structure of a separation branch channel portion 33 of the pretreatment integrated channel ver. 2-1 shown in FIG. 22 in Example 8.
FIG. 24 shows the degree of lysis (nucleic acid recovery rate) by three kinds of pretreatment integrated channels in Example 8.
FIG. 25 shows structures of three kinds of pretreatment integrated channels and complex formation rates by these pretreatment integrated channels in Example 8.
FIG. 26 shows an integrated channel in Example 8.
FIG. 27 shows an integrated device and detection results (Duration time) of Bacillus cereus using the device in Example 8.
FIG. 28 shows detection results (Duration time) of Bacillus cereus using an integrated device in Example 8.
FIG. 29 shows detection results (Duration time) of Bacillus cereus using an integrated device in Example 8.
FIG. 30 shows detection results (Duration time) of Bacillus cereus using an integrated device in Example 8.
FIG. 31 is a plot diagram showing a detection result of Bacillus cereus using an integrated device in Example 9.
FIG. 32 illustrates detailed structures of Channel 6 and Channel A in Example 2.
FIG. 33 illustrates detailed structures of Channel H and Channel I in Example 2.
FIG. 34 illustrates detailed structures of Channel G and Channel J in Example 2.
FIG. 35 illustrates detailed structures of Channel C and Channel D in Example 2.

### Description of Embodiments

### [Device]

A device of one embodiment is a microfluidic device 1 for detecting microorganisms in a sample, and includes a lysis section 2 for dissolving microorganisms and a separation section 3 for separating out a nucleic acid from a lysate of the microorganism formed in the lysis section communicating with the lysis section 2. Here, the lysis section 2 includes a channel for holding a bacteriolytic agent for lysing microorganisms, and the separation section 3 includes a means capable of separating out a nucleic acid contained in the lysate from other substances. The microfluidic device 1 may further include a complex forming section 4, and may further include a detection section 5.

A test sample may be a sample that may contain any microorganism, and examples thereof include various food samples, various water samples, various reagent samples, various media samples, and biological samples such as saliva, urine, and sweat. The sample may be liquid or solid, and is preferably liquid. An input amount of the sample is not particularly limited, and may be, for example, 1 µl to 100 ml, and is preferably 1 µl to 10 ml, more preferably 5 µl to 5 ml, and particularly preferably 50 to 500 µl, in the case of a liquid.

The microorganism that can be detected is not particularly limited, and examples thereof include various harmful lactic acid bacteria (for example, lactic acid bacteria included in the genus Lactobacillus, the genus Streptococcus, and the genus Lactococcus), yeasts (for example, film forming yeasts and off-flavor causing yeasts such as Brettanomyces), molds (for example, molds included in genus Penicillium and the genus Eurotium), other bacteria (for example, bacteria included in the genus Bacillus, the genus Campylobacter, the coliform group, the genus Staphylococcus, the genus Salmonella, the genus Listeria, the genus Mycoplasma, the genus Legionella, the genus Clostridium, and the genus Vibrio), drug resistant bacteria (for example, methicillin-resistant Staphylococcus aureus, vancomycin-resistant Staphylococcus aureus, and penicillin-resistant Streptococcus pneumoniae), and viruses (for example, norovirus and coronavirus), from the viewpoint of food hygiene and contact infection. In addition, a detection target is not limited to a microorganism, and may be any sample that can contain a nucleic acid. A lower limit of the detection of microorganisms may vary depending on the kind of microorganisms and detection means, and may be, for example, 100 to 1000 cfu/ml, and is preferably 10 to 100 cfu/ml.

The bacteriolytic agent is not particularly limited, and examples thereof include surfactants capable of dissolving lipids of cell membranes of microorganisms. Examples of the surfactant include: anionic surfactants such as alkyl sulfate ester salts (for example, dodecyl sulfate, octyl sulfate, decyl sulfate, tetradecyl sulfate, hexadecyl sulfate, and octadecyl sulfate), alkyl benzene sulfonates (for example, 4-octylbenzenesulfonate, 4-decylbenzenesulfonate, 4-dodecylbenzenesulfonate, 4-tetradecylbenzenesulfonate, 4-hexadecylbenzenesulfonate, and 4-octadecylbenzenesulfonate), α-olefin sulfonates, alkane sulfonates (for example, nonanesulfonate, decanesulfonate, undecanesulfonate, dodecanesulfonatesulfonate, tetradecanesulfonate, hexadecanesulfonate, and octadecanesulfonate), fatty acid salts (for example, caprylate, pelargonate, caprate, laurate, myristate, pentadecylate, palmitate, margarate, stearate, and arachidate), acyl sarcosinates (for example, octoyl sarcosinate, dodecanoyl sarcosinate, and decanoyl sarcosinate), cholates, and deoxycholates; cationic surfactants such as quaternary ammonium salts (for example, decyltrimethylammonium salts, dodecyltrimethylammonium salts, tetradecyltrimethylammonium salts, hexadecyltrimethylammonium salts, octadecyltrimethylammonium salts, and eicosyltrimethylammonium salts), pyridinium salts (for example, 1-decylpyridinium salts, 1-dodecylpyridinium salts, 1-tetradecylpyridinium salts, 1-hexadecylpyridinium salts, N-cetyl-2 methylpyridinium salts, N-cetyl-3 methylpyridinium salts, N-cetyl-4 methylpyridinium salts, 1-octadecylpyridinium salts, and 1-eicosylpyridinium salts), phosphonium salts (for example, tetraethylphosphonium salts, tributylmethylphosphonium salts, tetrabutylphosphonium salts, tetra-n-octylphosphonium salts, tributyldodecylphosphonium salts, tributylhexadecylphosphonium salts, methyltriphenylphosphonium salts, and tetraphenylphosphonium salts), dialkyl dimethyl ammonium salts, and alkyl dimethyl benzyl ammonium salts; amphoteric surfactants such as alkyl dimethylamine oxide and alkyl carboxybetaine; and nonionic surfactants such as polyoxyethylene alkyl phenyl ether (for example, polyoxyethylene (10) octylphenyl ether (Triton X-100)), polyoxyethylene alkyl ether, polyoxyethylene sorbitan ester (for example, polyoxyethylene (20) sorbitan monolaurate (Tween 20)), fatty acid sorbitan ester, alkyl monoglyceryl ether, fatty acid diethanolamide, and alkyl polyglucoside. Examples of counter ions of the anionic surfactant or the amphoteric surfactant include cations such as a sodium ion, a potassium ion, a lithium ion, a magnesium ion, a calcium ion, an iron ion, a silver ion, a copper ion, a nickel ion, a zinc ion, and a cobalt ion, and examples of the dodecyl sulfate include sodium dodecyl sulfate (SDS). Examples of counter ions of the cationic surfactant or the amphoteric surfactant include anions such as a fluoride ion, a chloride ion, a bromide ion, an iodide ion, and a hydroxide ion, and examples of the quaternary ammonium salt include dodecyltrimethylammonium bromide (DTAB). The amphoteric surfactant may form a salt with an anion and a cation contained in the same molecule. A concentration of a surfactant to be used as the bacteriolytic agent is not particularly limited as long as it is a concentration at which lipids of a cell membrane can be dissolved, and may be, for example, 0.001% (w/v) to 10% (w/v), and is preferably 0.01% (w/v) to 1% (w/v).

In addition to the surfactant, the bacteriolytic agent may further contain an enzyme capable of decomposing proteins in microbial cells. The enzyme is not particularly limited, and examples thereof include protease K, papain, ginger protease, trypsin, chymotrypsin, pepsin, pancreatin, elastase, and lysozyme.

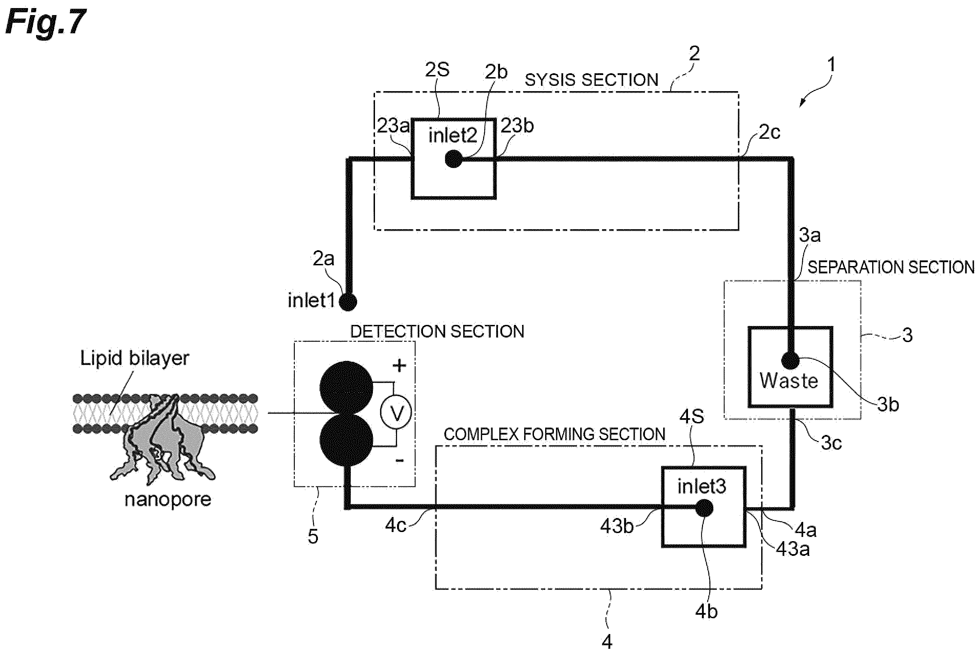

The device 1 of one embodiment includes the lysis section 2 and the separation section 3 provided in a microchannel. A microfluidic device 1 according to another embodiment includes a lysis section 2, a separation section 3, a complex forming section 4, and a detection section 5 provided in a microchannel (FIG. 7).

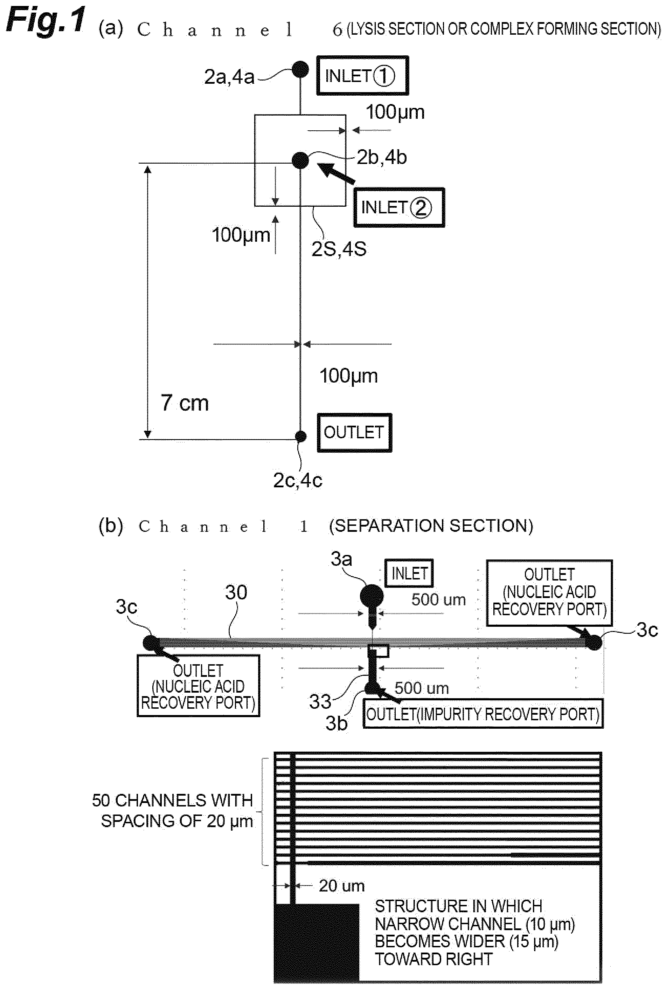

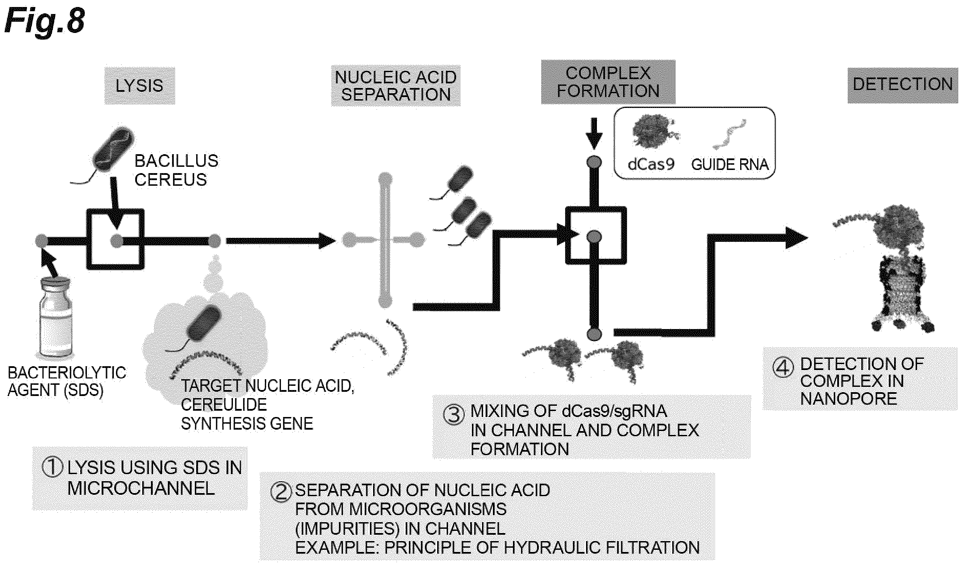

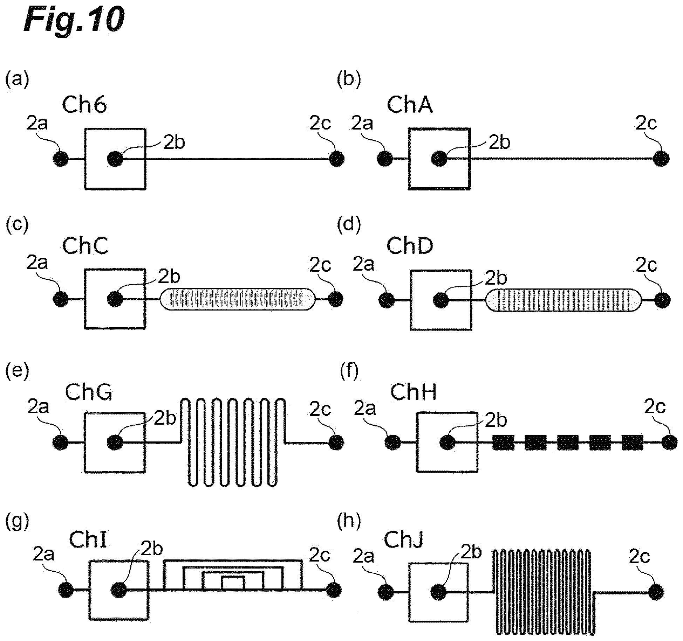

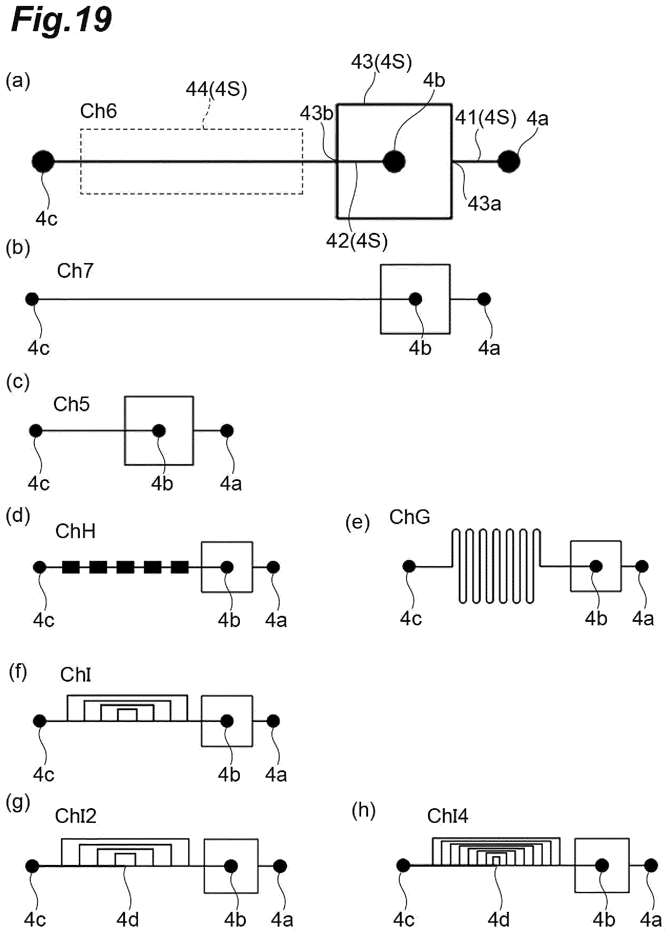

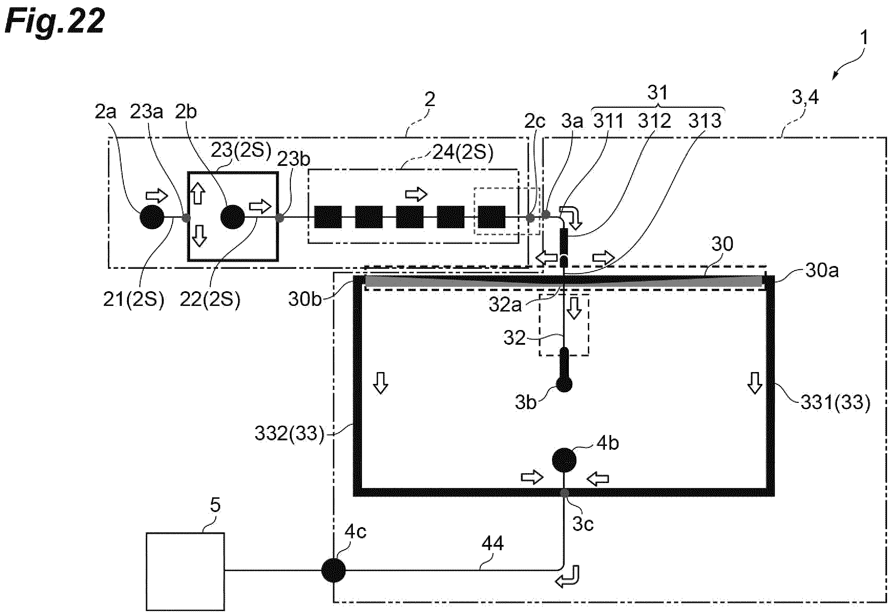

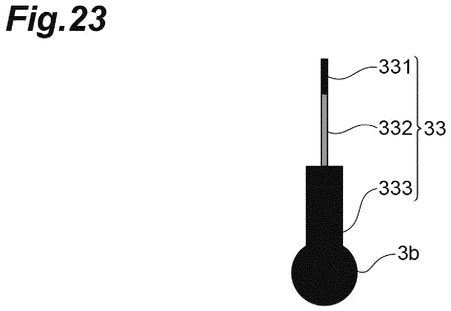

Hereinafter, the microfluidic device 1 according to one embodiment will be described with reference to FIGS. 7 and 8. In the following description, components denoted by reference numerals not shown in FIGS. 7 and 8 are shown in FIGS. 22 and 23 which illustrate a part of Example 8 in the microfluidic device 1 shown in FIG. 7 in detail. In a device main body 1, a microchannel is provided, a part of the microchannel is provided with the lysis section 2, the separation section 3, the complex forming section 4, and the detection section 5, and a sample is introduced from an inlet connected to the lysis section, that is, a sample receiving inlet 2a (inlet 1), flows to a bacteriolytic agent receiving inlet 2b (inlet 2), and passes through a channel for holding a bacteriolytic agent for lysing microorganisms provided in the lysis section 2, whereby the microorganisms are lysed while passing through the channel together with the bacteriolytic agent. The obtained microbial lysate (lysate solution) exits from a lysate solution discharging outlet 2c, enters the separation section 3 from the inlet 3a of the separation section, and is separated into a nucleic acid and other substances (cell membrane debris, impurities such as proteins; Waste) by a separation means provided in the separation section 3, and the nucleic acid further flows to the complex forming section 4 from an inlet of the complex forming section, that is, a first result receiving inlet 4a (inlet 3). The nucleic acid is formed into a protein-nucleic acid complex by passing through a channel holding a substance capable of forming a protein-nucleic acid complex (also referred to as a "complex forming substance") provided in the complex forming section 4, and finally flows to the detection section 5, and the presence or absence of a specific nucleic acid is detected. Note that the channel (the channels from 2a to 2c) for holding the bacteriolytic agent in the lysis section 2 and the channel (the channels from 4a to 4c) for holding the complex forming substance in the complex forming section 4 may have the same channel structures or different channel structures, and may be, for example, Channel 6 shown in (a) of FIG. 1 or have various structures shown in FIGS. 10 and 19.

A material of the microfluidic device main body is not particularly limited, and examples thereof may include a plastic resin such as polydimethylsiloxane (PDMS), but are not limited thereto. A shape of the microfluidic device is also not limited, and a size thereof may be a normal size as a microfluidic device, and is not particularly limited. A diameter of the microchannel connecting the lysis section, the separation section, the complex forming section, and the detection section is also not particularly limited, and may be, for example, 20 µm to 15 mm, and is preferably 100 µm to 0.5 mm.

### <Lysis Section>

The lysis section 2 performs a treatment of dissolving microorganisms contained in a sample by a bacteriolytic agent. By this treatment, microorganisms are lysed, and a lysate solution in which nucleic acids contained in microbial cells are dissolved can be obtained. The lysis time in the lysis section may be any time as long as the microorganisms are lysed, and may be, for example, 1 minute to 8 hours, and is preferably 5 minutes to 30 minutes.

The lysis section 2 has the sample receiving inlet 2a (inlet 1), the bacteriolytic agent receiving inlet 2b (inlet 2), and the lysate solution discharging outlet 2c. Further, the lysis section 2 has a lysis channel 2S connecting the inlets 2a and 2b and the outlet 2c. The lysis channel 2S includes a first lysis channel portion 21, a second lysis channel portion 22, a lysis branch channel portion 23, and a lysis mixing channel portion 24.

The first lysis channel portion 21 is a channel from the inlet 2a to a lysis branch part 23a. The lysis branch part 23a is a portion where the sample provided from the first lysis channel portion 21 is distributed and provided to the lysis branch channel portion 23. The second lysis channel portion 22 is a channel from the inlet 2b to a lysis confluence part 23b.

The lysis branch channel portion 23 extends along an outer peripheral side of a rectangular region in plan view. For example, the lysis branch channel portion 23 surrounds the inlet 2b and the second lysis channel portion 22. The above-described lysis branch part 23a may be defined as a part of the lysis branch channel portion 23. The lysis confluence part 23b is set on a side opposite to one side of the lysis branch channel portion 23 including the lysis branch part 23a. An end portion of the lysis mixing channel portion 24 and an end portion of the second lysis channel portion 22 are connected to the lysis confluence part 23b. At the lysis confluence part 23b, the sample provided from the lysis branch channel portion 23 and the bacteriolytic agent provided from the second lysis channel portion 22 are joined, and the joined material is provided to the lysis mixing channel portion 24. That is, the end portions of the lysis branch channel portion 23, the second lysis channel portion 22, and the lysis mixing channel portion 24 are connected to the lysis confluence part 23b.

The lysis mixing channel portion 24 is a portion for mixing and lysing the sample coming from the lysis branch channel portion 23 and the bacteriolytic agent coming from the inlet 2b. When the sample and the bacteriolytic agent are sufficiently mixed in the lysis mixing channel portion 24, a lysis rate increases, whereby the detection accuracy also increases.

In addition, the design of the channel 2S of the lysis section 2 is not particularly limited, but it is possible to increase the lysis rate of a channel by designing the two liquids, the bacteriolytic agent and the sample (for example, a bacterial suspension) to be actively mixed without forming a laminar flow, for example, by disposing protrusions/obstacles in the lysis mixing channel portion 24, extending the lysis mixing channel portion 24 as much as possible, mixing fine particles with a mixed liquid of the bacteriolytic agent and the sample, increasing a channel width ratio immediately before the two liquids meet, that is, the width ratio of a confluence channel in the lysis confluence part 23b, and the like. The lysis rate in the lysis section 2 may be 50% or higher (viability rate is lower than 50%), 60% or higher (viability rate is lower than 40%), 70% or higher (viability rate is lower than 30%), 80% or higher (viability rate is lower than 20%), or 90% or higher (viability rate is lower than 10%), and the viability rate can be measured, for example, by a method shown in Examples. The degree of lysis can also be calculated (defined) by a nucleic acid recovery rate. The nucleic acid recovery rate can be measured, for example, by a method described in Examples.

In order to obtain a desired mixing state and a desired lysis rate, for example, it is conceivable to change a flow state (pressure or speed) in the lysis mixing channel portion 24. That is, the lysis mixing channel portion 24 may be configured to change the flow state (pressure or speed) in the channel. Examples of such a configuration include a configuration in which a portion having a large channel area and a portion having a small channel area are alternately arranged, as shown in FIGS. 10(c), 10(d), and 10(f). Also, examples of the configuration for changing the flow state (pressure or speed) include a configuration for changing a flow direction (FIG. 10(e) or 10(h)) or a configuration including a branch part (FIG. 10(g)).

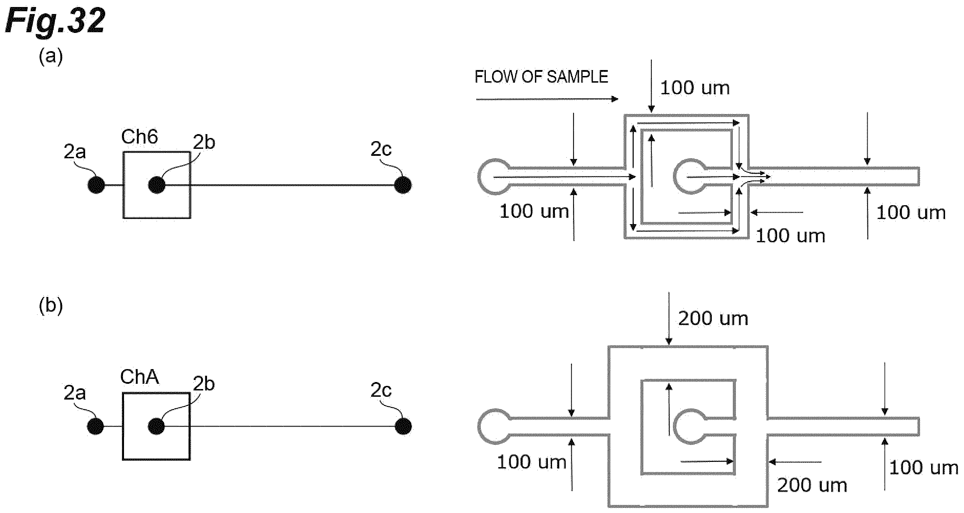

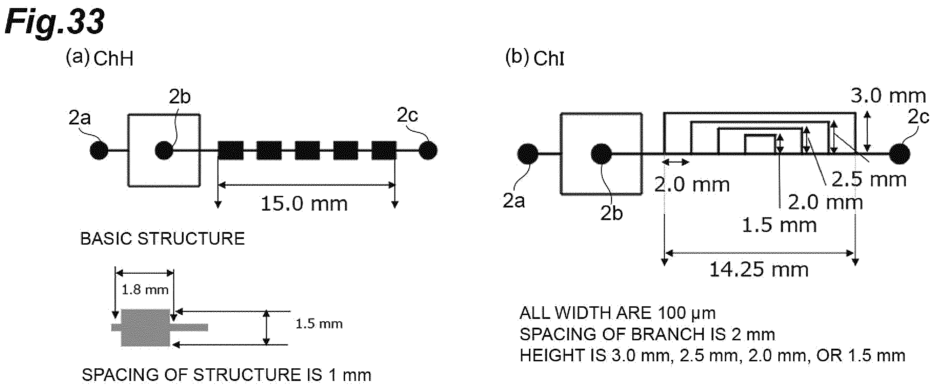

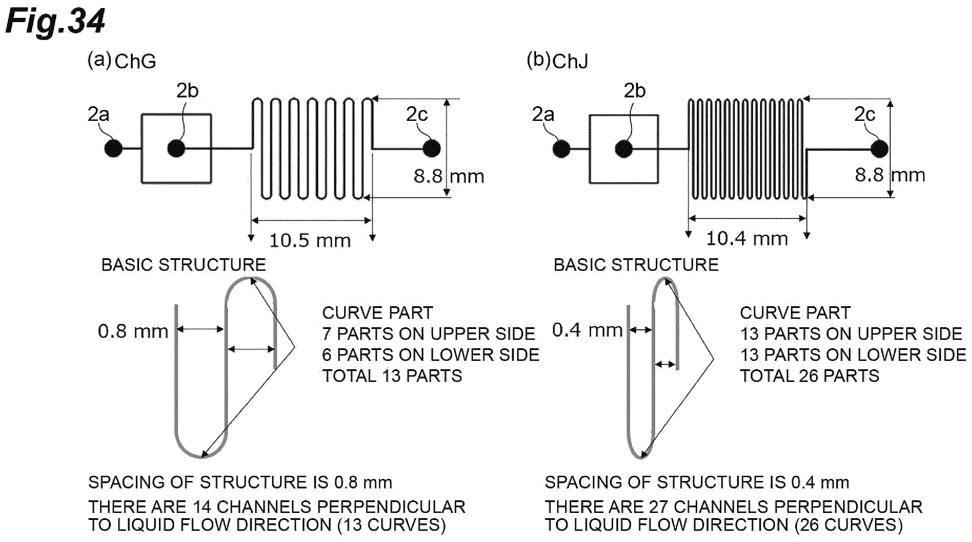

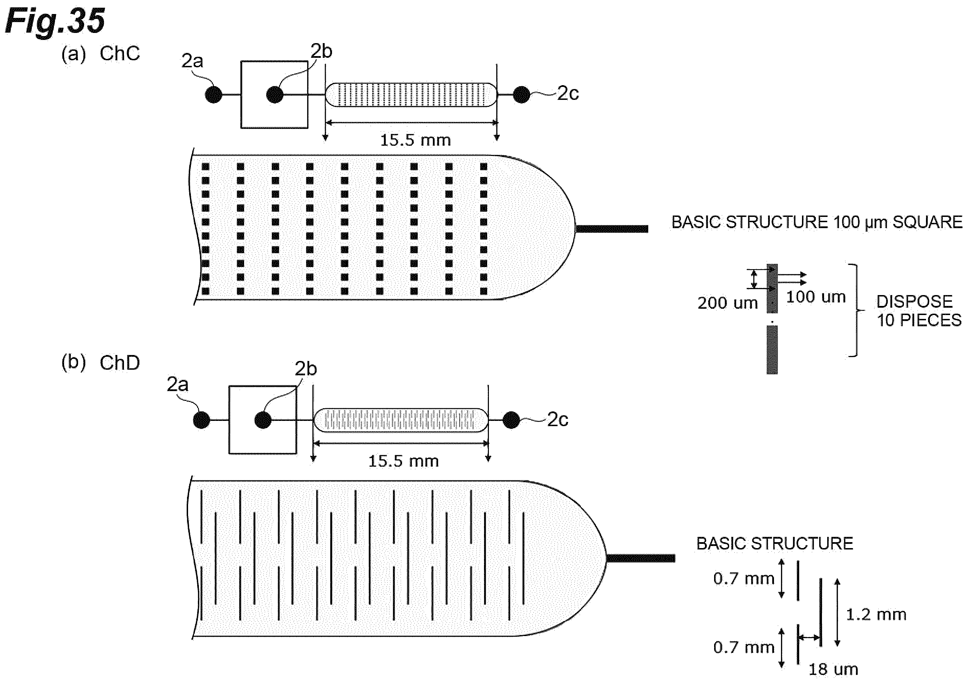

The width of the lysis channel 2S may be the same as a whole, and the width of the channel may be changed in order to increase the lysis rate. The width of the channel 2S is not particularly limited, and may be, for example, 10 µm to 1000 µm, 50 µm to 500 µm, 50 µm to 200 µm, or 100 µm to 200 µm. In addition, the length of the lysis channel 2S is not particularly limited, and the shorter the length of the portion excluding the lysis mixing channel portion 24, the smaller the loss of the sample and the higher the detection accuracy. On the other hand, the lysis mixing channel portion 24 may have a length that allows the bacteriolytic agent and the sample to be sufficiently mixed, and may be, for example, 1 mm or more, 3 mm or more, 5 mm or more, 7 mm or more, or 10 mm or more. On the other hand, when the length of the lysis mixing channel portion 24 is too long, sample loss occurs, and therefore the length may be 500 mm or less, 400 mm or less, 300 mm or less, 200 mm or less, 100 mm or less, or 50 mm or less. Specific examples thereof include the width and the length illustrated in FIGS. 32 to 35.

### <Separation Section>

The separation section 3 receives a lysate solution from the lysis section 2. The separation section 3 separates nucleic acid and impurities contained in the lysate solution. For example, the separation section 3 may separate and remove impurities from the lysate solution. The separation section 3 provides a result (liquid containing a nucleic acid) obtained by separating and removing impurities from the lysate solution to the complex forming section 4 provided on a downstream side.

The separation means in the separation section 3 is not particularly limited as long as it is a means capable of separating a nucleic acid and a substance other than the nucleic acid (non-nucleic acid substances, sometimes referred to herein as "impurity"), and examples thereof include a separation means using molecular size, a separation means using centrifugation, or a separation means using hydraulic filtration, and among them, a separation means using hydraulic filtration is preferable. In the hydraulic filtration, the size of a cell or a substance to be selected can be adjusted as desired by designing a channel by regarding a microchannel structure as an analog of a resistance circuit such as an electric circuit. For example, in a case where a microbial suspension or a lysate solution is caused to flow, since the degree of separation varies depending on the size and shape of the microorganisms, and the state of aggregation of the microorganisms, morphological information of the microorganisms can be obtained from the degree of separation in the channel.

When separation using hydraulic filtration is performed, the following channel design is performed. A plurality of secondary channels capable of drawing a constant flow rate are designed on both sides of a main channel which is the main. When the liquid flowing through the main channel is drawn into the secondary channel, the cells and fine particles flowing through the main channel are gradually pressed against the wall of the main channel. When the amount to be drawn into the secondary channel is determined, the water flowing through each of the main channel and the secondary channel is calculated as the width ratio of the main channel. When particles flowing near the wall are theoretically contained in half or more of the flow of water to be drawn into the secondary channel, that is, when the water to be drawn into the secondary channel is visualized, if the distance from the wall of the main channel exceeds a radius of the cells or the fine particles, these are drawn into the secondary channel. Depending on the width of the secondary channel and the number of stages, it is possible to change the flow rate drawn into the secondary channel, thereby controlling the size of cells and fine particles remaining in the main channel. By using such a separation theory, microorganisms whose membranes have been damaged by a bacteriolytic agent (cell debris and the like) are caused to flow into the main channel because of relatively large size thereof, whereas small molecules such as nucleic acids contained in the microorganisms are caused to flow into the secondary channel (Masumi Yamada, Minoru Seki, Hydrodynamic filtration for on-chip particle concentration and classification utilizing microfluidics, Lab on a Chip, 2005, 5.11: 1233-1239.). In the present invention, the nucleic acid flowing into the secondary channel is used as a subject to be measured.

The separation section 3 of one embodiment includes an inlet 3a, an impurity recovery port 3b, an outlet 3c, and a separation unit 30. The separation unit 30 to which the lysate solution has been provided from the inlet 3a discharges the first result containing a nucleic acid from the outlet 3c and discharges a second result containing impurities from the impurity recovery port 3b. The second result contains impurities having large molecular sizes and may also contain a nucleic acid. The first result contains a highly pure nucleic acid and may contain a small amount of impurities, but it is preferable that the first result does not contain impurities. The nucleic acid recovery rate of the separation section 3 can be defined as a ratio of the amount of nucleic acid in the first result recovered from the outlet 3c to the amount of nucleic acid in the lysate solution charged into the inlet 3a (nucleic acid recovery rate), and the nucleic acid recovery rate of the separation section 3 may be 50% or higher, 60% or higher, 70% or higher, 80% or higher, or 90% or higher, and the nucleic acid recovery rate can be measured, for example, by a method shown in Examples.

The separation section 3 includes a first separation channel portion 31, a second separation channel portion 32, and a separation branch channel portion 33. The first separation channel portion 31 connects the inlet 3a to the separation unit 30. In the separation unit 30, a position at which the first separation channel portion 31 is connected is substantially the center in an extending direction of the separation unit 30. Note that the inlet 3a does not need to be a physical component. The inlet 3a is a portion defined as a portion for receiving the lysate solution. In addition, the outlet 2c connected to the inlet 3a is a portion defined as a portion for discharging the lysate solution to the inlet 3a, and does not need to be a physical component, like the inlet 3a.

In one embodiment (FIG. 22), the first separation channel portion 31 may include a first small diameter channel part 311, a large diameter channel part 312, and a second small diameter channel part 313. For example, a diameter of the large diameter channel part 312 may be about 5 times larger than that of the first small diameter channel part 311. The first small diameter channel part 311 is disposed on an inlet 3a side, and the second small diameter channel part 313 is disposed on an outlet 3c side. That is, the second small diameter channel part 313 is connected to the separation unit 30. The large diameter channel part 312 is disposed between the first small diameter channel part 311 and the second small diameter channel part 313.

The second separation channel portion 32 connects the output of separation unit 30 to outlet 3c. In the separation unit 30, a position at which the second separation channel portion 32 is connected is substantially the center in the extending direction of the separation unit 30. That is, the first separation channel portion 31 and the second separation channel portion 32 may be arranged in a straight line on a certain imaginary line. The second result containing impurities is discharged to the impurity recovery port 3b. The impurity recovery port 3b includes a chamber having a volume capable of pooling the second result.

FIG. 23 is an enlarged view of an example of the second separation channel portion 32. The second separation channel portion 32 includes a small diameter channel part 321, a first large diameter channel part 322, and a second large diameter channel part 323. An input end of the small diameter channel part 321 is connected to the separation unit 30. An output end of the second large diameter channel part 323 is connected to the impurity recovery port 3b. The first large diameter channel part 322 is disposed between the small diameter channel part 321 and the second large diameter channel part 323. A channel diameter of the first large diameter channel part 322 is larger than a channel diameter of the small diameter channel part 321 but smaller than a channel diameter of the second large diameter channel part 323. That is, in the second separation channel portion 32, the channel diameter gradually increases as approaching the impurity recovery port 3b. For example, a diameter of the small diameter channel part 321 may be 20 µm, a diameter of the first large diameter channel part 322 may be 25 µm, and a diameter of the second large diameter channel part 323 may be 500 µm. Also, the length of the small diameter channel part 321 is longer than the length of the first large diameter channel part 322. For example, the length of the small diameter channel part 321 is 1 cm, and the length of the first large diameter channel part 322 is 3.9 mm.

The separation branch channel portion 33 connects the first part 30a that outputs the first result from the separation unit 30 and the second part 30b that outputs the first result, to the outlet 3c. The separation branch channel portion 33 has a U shape in plan view. The separation branch channel portion 33 includes the outlet 3c which is a confluence part connected to an end of the inlet 4a of the complex forming section. The outlet 3c may be disposed substantially at the center of one side of the separation branch channel portion 33. As a result, a first path 331 from the separation unit 30 to the outlet 3c and another second path 332 from the separation unit 30 to the outlet 3c can have the same channel length.

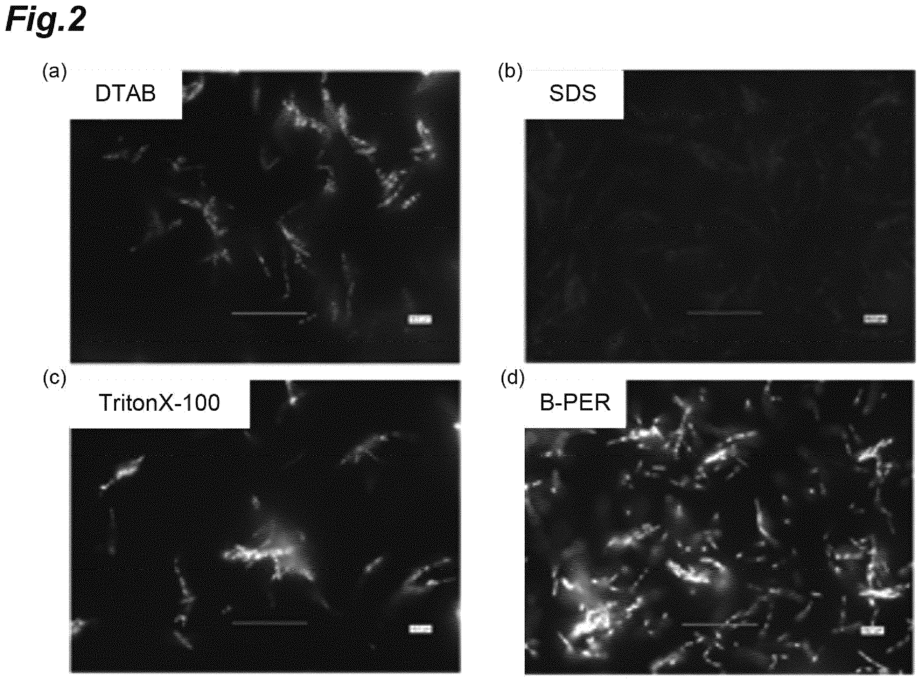

The outlet 3c is connected to the complex forming section 4. In one embodiment, the outlet 3c is connected to a complex branch channel portion 43 (FIG. 19(a)). In another embodiment, the separation branch channel portion 33 can also serve as the complex branch channel portion 43 that is a part of a complex channel 4S which will be described later. In this case, a first complex channel portion 41, a second complex channel portion 42, and the complex branch channel portion 43 can be omitted, and the outlet 3c is connected to a complex mixing channel portion 44 (FIG. 22). In this case, 3c in FIG. 22 may be used as the outlet 3c of the separation section 3, and 30a and 30b in FIG. 2 may be used as the outlet 3c of the separation section 3 (FIG. 1(b)). In the latter case, the separation branch channel portion 33 in FIG. 22 can be used as the complex branch channel portion 43, and the outlet 3c in FIG. 22 can be used as 43b.

### <Complex Forming Section>

The complex forming section 4 reacts the first result with a complex forming substance to form a complex. In the complex forming section 4, a microchannel holding a specific nucleic acid in the nucleic acid (nucleic acid derived from microorganisms) in the first result separated in the separation section 3 and a substance capable of forming a protein-nucleic acid complex (complex forming substance) is provided.

Examples of the complex forming substance include CRISPR-dCas9 to which sgRNA specific to the specific nucleic acid is bound, and a protein marker that specifically binds to the specific nucleic acid. The specific nucleic acid (in the present specification, may be referred to as a "target nucleic acid") is preferably a gene or a part thereof specific to the microorganism species or the microorganism genus, which can specify the microorganism species or the microorganism genus. For example, in a case where the microorganism is Bacillus cereus, the target nucleic acid may be a lecithinase gene or a cereulide synthesis gene, or a part thereof.

The time for forming the complex in the complex forming section 4 is not particularly limited, and may be, for example, 1 minute to 8 hours, and is preferably 5 minutes to 30 minutes. In addition, a channel design of the complex forming section is not particularly limited, but it is possible to increase the complex formation rate by designing the two liquids, a solution of the bacteriolytic agent and a solution of the complex forming substance to be actively mixed without forming a laminar flow as described above. That is, the complex forming section may have the above-described configuration that changes the flow state (pressure or speed). The complex formation rate may be 50% or higher, 60% or higher, 70% or higher, 80% or higher, or 90% or higher, and the complex formation rate can be measured, for example, by a method shown in Examples.

The target nucleic acid and CRISPR-dCas9 to which sgRNA specific to the target nucleic acid is bound can form a complex by Watson-Crick base pair DNA-RNA interaction. In addition, in order to detect the complex, labeling may be performed (for example, Patent Literature 3). The length of the sgRNA is not particularly limited, and is preferably 20 residues or more. Furthermore, the sgRNA may be a single molecule, and may be, for example, two or more molecules such as a complex of CRISPR RNA (crRNA) and trans-activating CRISPR RNA (tracrRNA), which are homing devices originally possessed by bacteria and archaea. Examples of the complex include a complex of Cas9 and sgRNA in which nuclease and nickase activities completely disappear due to introduction of mutation into D10A and H840A, deletion of an active site, or the like, and a complex of Cas9 and tracrRNA or crRNA. Examples of the Cas protein include Cas9 classified into the type II CRISPR/Cas system, and also include a crRNA-Cascade-Cas3 complex, a crRNA-Csm (III-A) or a Cmr (III-B) complex of the type I or type III CRISPR-Cas system, and a Cas12f (Cpf1)-crRNA complex of the type V CRISPR-Cas system. Furthermore, examples thereof include a crRNA-Csf2-Cas11 complex of type IV CRISPR-Cas system. Examples thereof include a crRNA-Cas13 of the type VI CRISPR-Cas system targeting RNA sequences. The origin of the Cas protein described above is not particularly limited.

The protein marker that specifically binds to the target nucleic acid is preferably one that can bind to the target nucleic acid by a secondary structure of a protein such as a helix recognizing a nucleic acid sequence. Examples of the protein marker include Transcription activator-like effector (TALE), Zinc-Finger (ZF), and Pentatricopeptide repeat (PPR) protein.

The complex forming section 4 may have a structure similar to that of the lysis section 2 (FIGS. 1 and 19). The complex forming section 4 of one embodiment has a first result receiving inlet 4a, a complex forming substance receiving inlet 4b (inlet 3), and a complex discharging outlet 4c. Further, the complex forming section 4 has the complex channel 4S connecting the inlets 4a and 4b and the outlet 4c. The complex channel 4S includes a first complex channel portion 41, a second complex channel portion 42, a complex branch channel portion 43, and a complex mixing channel portion 44.

The first complex channel portion 41 is a channel from the inlet 4a to a complex branch part 43a. The complex branch part 43a is a portion where the first result provided from the first complex channel portion 41 is distributed and provided to the complex branch channel portion 43. The second complex channel portion 42 is a channel from the inlet 4b to a complex confluence part 43b.

The complex branch channel portion 43 extends along an outer peripheral side of the rectangular region in plan view. For example, the complex branch channel portion 43 surrounds the inlet 4b and the second complex channel portion 42. The above-described complex branch part 43a may be defined as a part of the complex branch channel portion 43. The complex confluence part 43b is set on a side opposite to one side of the complex branch channel portion 43 including the complex branch part 43a. An end of the complex mixing channel portion 44 and an end of the second complex channel portion 42 are connected to the complex confluence part 43b. In the complex confluence part 43b, the sample provided from the complex branch channel portion 43 and the complex forming substance provided from the second complex channel portion 42 are joined, and the joined material is provided to the complex mixing channel portion 44. That is, the end portions of the complex branch channel portion 43, the second complex channel portion 42, and the complex mixing channel portion 44 are connected to the complex confluence part 43b.

The complex mixing channel portion 44 is a portion for mixing the first result coming from the complex branch channel portion 43 and the complex forming substance coming from the inlet 4b to form a complex. When the first result and the complex forming substance are sufficiently mixed in the complex mixing channel portion 44, the complex formation rate increases, whereby the detection accuracy also increases.

The design of the complex channel 4S of the complex forming section 4 is not particularly limited, and may be the same as the design of the channel 2S of the lysis section 2 (FIGS. 10 and 19). The complex formation rate in the complex forming section 4 may be 40% or higher, 50% or higher, 60% or higher, 70% or higher, 80% or higher, or 90% or higher, and the complex formation rate can be measured, for example, by a method shown in Examples.

In order to obtain a desired mixing state and a desired complex formation rate, for example, it is conceivable to change a flow state (pressure or speed) in the complex mixing channel portion 44. That is, the complex mixing channel portion 44 may be configured to change the flow state (pressure or speed) in the channel. Examples of such a configuration include a configuration in which a portion having a large channel area and a portion having a small channel area are alternately arranged, as shown in FIG. 19(d). Also, examples of the configuration for changing the flow state (pressure or speed) include a configuration for changing a flow direction (FIG. 19(e)) or a configuration including a branch part (FIGS. 10(f), 10(g), and 10(h)).

The width of the complex channel 4S may be the same as a whole, and the width of the channel may be changed in order to increase the complex formation rate. The width of the complex channel 4S is not particularly limited, and may be, for example, 10 µm to 1000 µm, 50 µm to 500 µm, 50 µm to 200 µm, or 100 µm to 200 µm. In addition, the length of the complex channel 4S is not particularly limited, and the shorter the length of the portion excluding the complex mixing channel portion 44, the smaller the loss of the sample and the higher the detection accuracy. On the other hand, the complex mixing channel portion 44 may have a length that allows the first result and the complex forming substance to be sufficiently mixed, and may be, for example, 1 mm or more, 3 mm or more, 5 mm or more, 7 mm or more, or 10 mm or more. On the other hand, when the length of the complex mixing channel portion 44 is too long, sample loss occurs, and therefore the length may be 500 mm or less, 400 mm or less, 300 mm or less, 200 mm or less, 100 mm or less, or 50 mm or less. Specific examples thereof include the width and the length illustrated in FIGS. 32 to 35.

### <Detection Section>

The detection section 5 including a means for detecting the complex is not particularly limited, and for example, preferably includes a means for electrochemically or optically detecting the complex, and particularly preferably detects the complex using nanopores. In addition, a means for detecting the nucleic acid by real-time PCR or an immunological method may be provided.

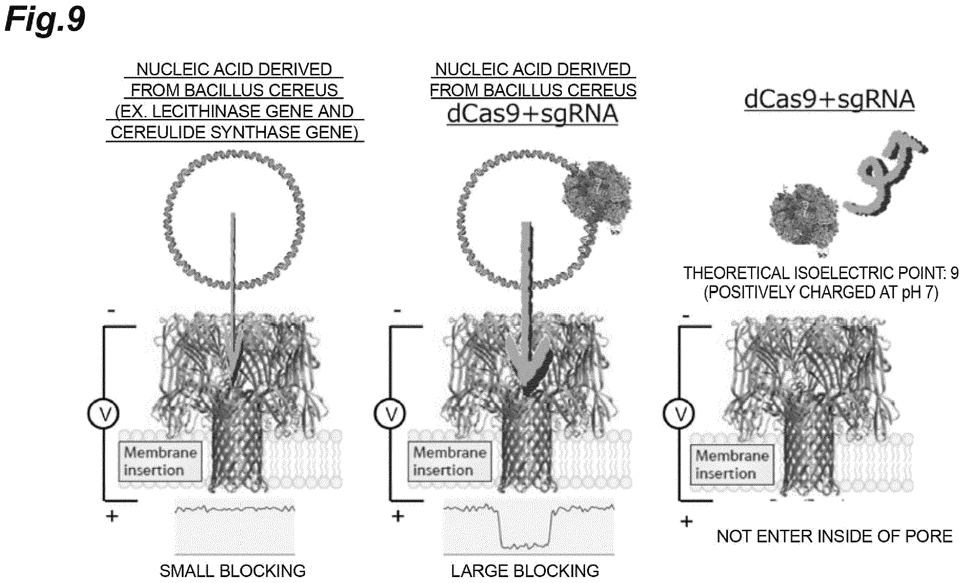

Regarding detection by nanopores, detection can be performed by, for example, a mechanism as described in Non Patent Literature 1. The nanopores are obtained by embedding a transmembrane protein on a lipid bilayer membrane, and the transmembrane protein has openings (nanopores). When a voltage is applied to both sides of the lipid bilayer membrane, a current flows. When the complex fits into an opening of the nanopore, a current value in the nanopore temporarily decreases (blocking), and when the complex passes through or kicked out of the nanopore, the current value recovers, so that the presence of the complex can be detected. On the other hand, in the case of the nucleic acid, since the size thereof is small, the time during which the nucleic acid stays in the nanopore is short, or the change in the current value when the nucleic acid passes through the nanopore is small (small blocking), so that the nucleic acid can be distinguished from the complex. On the other hand, in the case of CRISPR-dCas9 not bound to a nucleic acid, it is positively charged under the measurement pH condition and does not enter the inside of the nanopore (FIG. 9).

The technique used in Non Patent Literature 1 is called solid state nanopore (solid nanopore). The solid nanopore is formed by irradiating a solid material such as silicon nitride or graphene with an electron beam by a semiconductor microfabrication technique. Large scale devices are required to produce solid nanopores, and there remains a problem in the cost of pore production and reliability of pore diameter.

On the other hand, the nanopore used in the present invention is called a biological nanopore using a membrane protein. Since the biological nanopore is formed by spontaneous association and insertion of a membrane protein into the membrane, pore formation is easy, and a pore diameter can be adjusted by one molecular unit of protein by performing protein engineering modification. Therefore, a pore having a diameter corresponding to the size of the nucleic acid-protein complex as a target can be designed and constructed, and more sensitive detection becomes possible.

The method for adjusting the pore diameter is not particularly limited, but for example, in the case of a pore of a type in which a plurality of membrane protein monomers associate to form a pore, the number of monomers forming the pore can be controlled by eliminating or imparting a structural steric hindrance when the monomers interact with each other, and the pore diameter can be increased or decreased. In addition, the number of monomers forming the pore can be controlled by rigidizing the main chain of the monomer by amino acid mutation or reducing molecular fluctuation of the monomer by an experiment at a low temperature. In addition, pores having a predetermined diameter can be formed by creating pores of a desired diameter with DNA or the like and recruiting the membrane protein monomers thereof. Alternatively, pores having a predetermined diameter can be formed by introducing a random mutation of amino acids into a monomer and selecting a molecule that forms a pore having a predetermined diameter. The monomer is not limited to a pure membrane protein, and for example, a pore modified with a protein marker can be produced by binding and co-expressing the monomer and the protein marker. When the target nucleic acid is added to such a modified pore, the target nucleic acid can be detected by a decrease in the current value due to the binding between the target nucleic acid and the protein marker. Further, by selecting a protein marker having an optimum size for the pore whose diameter is adjusted, more sensitive detection can be achieved.

The size of the nanopore is not particularly limited as long as it is adjusted in accordance with the size of the complex, and may be, for example, 1 to 100 nm, and is preferably 15 nm to 40 nm.

Examples of the optical detection include fluorescence detection and absorption detection, and also include spectroscopic detection such as infrared spectroscopy, near-infrared spectroscopy, and Raman spectroscopy.

The microfluidic device 1 may be, for example, an integrated device in which a lysis section and a separation section are provided on a device substrate made of glass or a resin material, or may be an integrated device in which a lysis section, a separation section, and a complex forming section are provided, or an integrated device in which a lysis section, a separation section, a complex forming section, and a detection section are provided. Note that the microfluidic device 1 may include an additional device connected to the device substrate in order to perform the functions thereof. For example, the microfluidic device 1 may include a pump and a tube connected to the device substrate (specifically, an inlet and/or an outlet of each functional unit).

The microfluidic device 1 of one embodiment includes the lysis section 2, the separation section 3 communicating with the lysis section, the complex forming section 4 communicating with the separation section 3, and the detection section 5 communicating with the complex forming section 4. In this case, it is preferable that the detection section 5 includes a means for detecting the complex formed in the complex forming section 4, for example, a means for electrochemically or optically detecting the complex, particularly a detection means using a nanopore.

A microfluidic device 1 according to another embodiment includes a lysis section 2, a separation section 3 communicating with the lysis section, and a detection section 5 communicating with the separation section 3. That is, the complex forming section may not be provided. In this case, it is preferable that the detection section 5 includes a means for detecting the nucleic acid obtained in the separation section 3, for example, a detection means using real-time PCR or an immunological method.

### [Detection Method]

A method for detecting microorganisms in a sample according to one embodiment is a method using the microfluidic device of the present invention. In addition, it is preferable that the microfluidic device used be a device which has been integrated (integrated device).

The detection method of one embodiment includes a lysis step of lysing microorganisms in a sample in the lysis section; and a separation step of separating out a nucleic acid from a lysate of microorganisms, in the separation section. The detection method may further include a complex forming step of forming a protein-nucleic acid complex from the nucleic acid separated in the separation section in the complex forming section, and may further include a detection step of detecting the protein-nucleic acid complex formed in the complex forming section, in the detection section.

The method for detecting microorganisms in a sample of one embodiment uses the microfluidic device, and includes:
a step of lysing microorganisms to obtain a lysate of the microorganisms; and
a step of separating out a nucleic acid from the obtained lysate,
may further include:
   a step of forming a protein-nucleic acid complex from the separated nucleic acid, and
   may further include:
      a step of detecting the complex.

The detection method according to another embodiment includes: a lysis step of lysing microorganisms in a sample in the lysis section; a separation step of separating out a nucleic acid from a lysate of microorganisms obtained in the lysis section, in the separation section; and a detection step of detecting the nucleic acid separated in the separation section, in the detection section.

### Examples

### Example 1 Device Preparation and Microorganism Detection

### (Design of Channel and Creation of Photomask)

Design software Illustrator (manufactured by Adobe, Inc.) was used to design a channel. The designed channel (Channel 6 and Channel 1) is shown in FIG. 1. The Channel 6 has a width of 100 µm, and the detailed structure of the channel is the same as that in FIG. 32(a). Thereafter, a blueprint was sent to Tokyo Process Service (TOPRO) Co., Ltd. to request photomask production. The photomask was created such that a size was 4 inches, light-shielding/transmitting parts: a white part was light-shielding and a black part was transmitting, and a film surface: a printed surface appeared as designed when viewed from a glass side.

### (Creation of Mold for Microchannel)

A 4 inch Si wafer was placed in a plasma generator (Yamato Scientific Co., Ltd., PR-200) with a mirror surface facing upward, and treated at 100 W for 30 seconds. The plasma-treated wafer was set in a central portion of a spin coater, and SU-8 resist (SU-8 3025 for Channel 1 and SU-8 3050 for Channel 6) manufactured by Nippon Kayaku Co., Ltd. was added dropwise to the central portion of the wafer with a dropper whose tip was cut. The resist was spread over the entire surface of the wafer using a dropper, and then a spin coater was rotated to uniformly apply the resist. The number of revolutions was 500 rpm × 30 seconds, 500 → 4000 rpm (10 seconds), and 4000 rpm × 30 seconds. After incubation at 95°C for 30 minutes or more, the temperature was lowered to 65°C to solidify the resist. After the temperature was returned to room temperature, a film thickness was measured.

### (UV Exposure)

The mask was set on the Si wafer so that a Cr surface was in contact with the wafer, and exposed to light with a UV exposure device (manufactured by Nanotech Co., Ltd.) at 150 W for 5 seconds. After the exposure, the film was incubated again at 95°C for 30 minutes.

### (Development and Release Process)

The SU-8 Developer was placed in a petri dish, and the wafer was immersed and developed for 2 or 3 minutes while a liquid level was appropriately shaken. The wafer was lifted from the petri dish with tweezers, and a developer was poured on both front and back surfaces while being tilted. A compressor was applied on a Kimtowel to blow off a solution and let it dry well. Thereafter, a 1% solution of dimethyloctadecylchlorosilane (solvent: toluene) was prepared as a release treatment liquid, and the solution was placed in a petri dish, the wafer was immersed, and left for about 5 minutes. The wafer was washed with toluene, and then dried well by blowing off the solution with a compressor.

### (Preparation of PDMS)

The silicone potting material SILPOT^{™}184 (Dow Toray) Erastomer Base and Erastomer Curing Agent were weighed at a ratio of 10:1, and mixed. An adjustment liquid was poured onto the prepared base, placed in a desiccator, degassed, and then incubated at 85°C for 35 minutes. After the temperature was returned to room temperature, the periphery of the channel was cut out and further trimmed, then a hole was made with a biopsy trephine or a leather punch (2.0 mm), and the core was removed with tweezers. The cored PDMS and slide glass were placed in a plasma generator with the surfaces to be bonded facing up, treated at 100 W for 10 seconds, then the slide glass was taken out on the petri dish with tweezers, and the PDMS was bonded to the slide glass. A silicone tube having an appropriate diameter was inserted into an inlet and an outlet of the channel and subjected to evaluation.

### (Culture of Bacillus Cereus Strain)

As Bacillus cereus to be measured, Bacillus cereus JCM17690 strain (cereulide synthase gene-carrier strain) and NBRC3836 strain (cereulide synthase gene-non-carrier strain) were used. The glycerol stocks were streaked onto standard agar media and plated overnight at 30°C under aerobic conditions. The cells were planted in tryptic soy broth liquid medium and cultured overnight at 30°C with shaking. On the next day, the number of bacteria was estimated by colony count, and a dilution series of the culture solution was prepared with peptone added saline.

### (Selection of Bacteriolytic Agent)

The channel of Channel 6 was used for verification. The bacteriolytic agent and the microbial suspension (Bacillus cereus suspension suspended in 0.9% saline so as to have a bacterial concentration of 10⁷ cfu/ml) were each fed at 3.0 µl/min or 4.0 µl/min using a syringe pump. B-PER manufactured by Thermofisher, cationic dodecyltrimethylammonium bromide (DTAB, Tokyo Chemical Industry Co., Ltd.), nonionic TritonX-100 (Fujifilm Wako Pure Chemical Industries, Ltd.), and anionic sodium dodecyl sulfate (SDS, FUJIFILM Wako Pure Chemical Corporation) were added as a bacteriolytic agent so as to have a final concentration of 1%, the mixture was reacted at room temperature for 15 hours, then a nucleic acid was stained with GelRed manufactured by Biotium Inc., and observation was performed using an all-in-one fluorescence microscope BZ-X800 manufactured by Keyence Corporation. The results are shown in FIG. 2. With respect to DTAB and TritonX-100, the red fluorescence intensity (bright portion in FIG. 2) was reduced as compared with B-PER, but the fluorescence of red aggregates was still observed in the cells. On the other hand, regarding SDS, the red fluorescence intensity greatly decreased, suggesting that the internal nucleic acid efficiently diffuses to the outside of bacteria. From this result, it was considered that SDS as an anionic surfactant was suitable for nucleic acid extraction.

### (Verification of Bacillus Cereus Lysis Rate in Microchannel Using SDS)

Channel 6 as a lysis section (lysis channel) and Channel 1 as a separation section (separation channel) were used. SDS was used as a bacteriolytic agent. For the purpose of verifying the lysis rate in the presence of 0.05%, 0.1%, or 0.2% SDS, or in 0.1% SDS and 200 µg/ml proteinaze K (manufactured by FUJIFILM Wako Pure Chemical Corporation), or in 0.05% SDS and 200 µg/ml proteinase K, each of the bacteriolytic agents and the microbial suspension (Bacillus cereus suspension suspended in 0.9% saline so as to have a bacterial concentration of 10⁷ cfu/ml) were each delivered at 8.0 µl/min using a syringe pump to the channel of Channel 6. Thereafter, it was connected to an inlet of Channel 1 with a 1 mm diameter tube, and a sample was collected from the secondary channel outlet (nucleic acid recovery port).

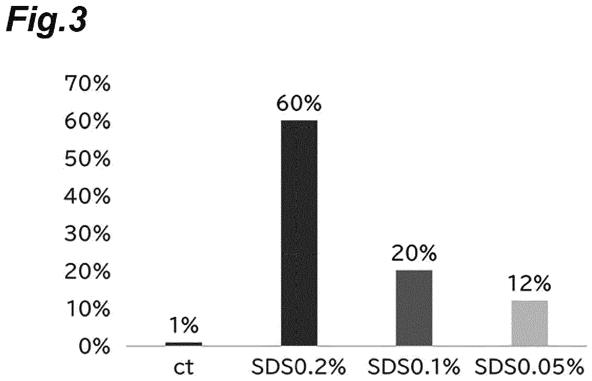

The lysis rate was calculated by quantitative PCR. As primers, enzymes, and the like, those attached to the CycleavePCRtm Bacillus cereus (CRS gene) Detection Kit (Takara Bio Inc.) were used. The lysate solution recovered from the secondary channel outlet was purified by QIAquick Gel Extraction Kit (manufactured by Qiagen) and verified under the following conditions. Hold (initial denaturation): 95°C, 10 s → 3 step PCR: 45 cycles (95°C, 5 s → 55°C, 10 s → 72°C, 20 seconds). A Ct value of the sample that was left standing overnight after addition of SDS to a bacterial fluid at the same concentration in vitro was regarded as a "100% lysed" control, and the lysis rate of the sample lysed in the channel under each SDS concentration condition was evaluated. The results are shown in FIG. 3. While 0.2% SDS had the highest lysis rate of 60%, 0.1% SDS and 0.05% SDS had a lysis rate of 20% or lower.

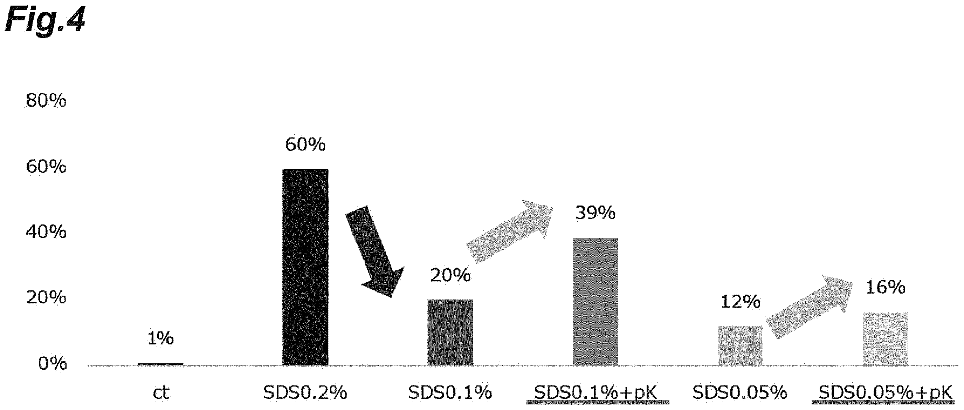

In the studies on nanopore detection so far, it was confirmed from experiments that "SDS at a certain concentration or higher inhibits the formation of lipid bilayer membranes", and it was predicted that it is necessary to secure the lysis rate even if the SDS concentration is lowered. Therefore, Proteinase K, which has been reported to enhance the lytic effect thereof in the presence of SDS (Mohammad, Shahriar et al. 2011 "Effect of Proteinase-K on Genomic DNA Extraction from Gram-Positive Strains." Stamford Journal of Pharmaceutical Sciences 4, no. 1: 53-57.), was added to verify the lysis rate thereof. FIG. 4 illustrates a verification result. By addition of proteinase K, the degree of lysis at the time of addition of 0.1% SDS was recovered to about 2/3. From this result, it was suggested that the lysis rate was improved by using SDS and proteinase K in combination as compared with SDS alone.

### (Verification of Complex Formation in Presence of SDS)

In integrating lysis, separation, complex formation, and detection, it was suggested that SDS used during lysis may inhibit the formation of a complex between dCas9/sgRNA and a target nucleic acid. Therefore, whether or not the complex can be formed at the SDS concentration contained in the sample passing through Channel 6 and Channel 1 was verified in vitro.

When 0.2% SDS was added to Channel 6, the SDS concentration after passing through Channel 6 and Channel 1 was calculated to be about 0.0225%. 3 µl of 1.5 µg/µl dCas9 NLS (manufactured by NIPPON GENE CO., LTD.) and 1.5 µl of 23µM sgRNA (targeting 5'-AGAACGAAUGCGAAAGAAGAUAGUUUUAGAGCUAGAAAUA GCAAGUUAAAAUAAGGCUAGUCCGUUAUCAACUUGAAAAA GUGGCACCGAGUCGGUGCUUUU-3' (SEQ ID NO. 1), and AACGAATGCGAAAGAAGATATGG (SEQ ID NO. 2) of cesA gene, adjusted with EnGen sgRNA Synthesis Kit manufactured by NEB) were mixed, and the mixture was left at room temperature for 5 minutes. Thereafter, 0.45 µl of 1% SDS, 5 µl of 89 ng/µl cesA plasmid prepared from a cereulide holding strain, 2 µl of 10× H buffer (manufactured by Takara), and 8.05 µl of MilliQ (trademark) water were added, and the mixture was incubated for 37°C for 60 minutes. Thereafter, 2.5 µl was taken, and 1 µl of a glycerol dye was added and mixed. The mixed liquid was electrophoresed at 4°C and 30 mA for 130 minutes using Super Sep-Ace 10% to 20% gel (FUJIFILM Wako Pure Chemical Corporation) and Tris-glycine buffer (pH 9.5). After electrophoresis, the cells were immersed in a 1× GelRed solution and stained for 20 minutes. After staining, images were taken with a UV imager.

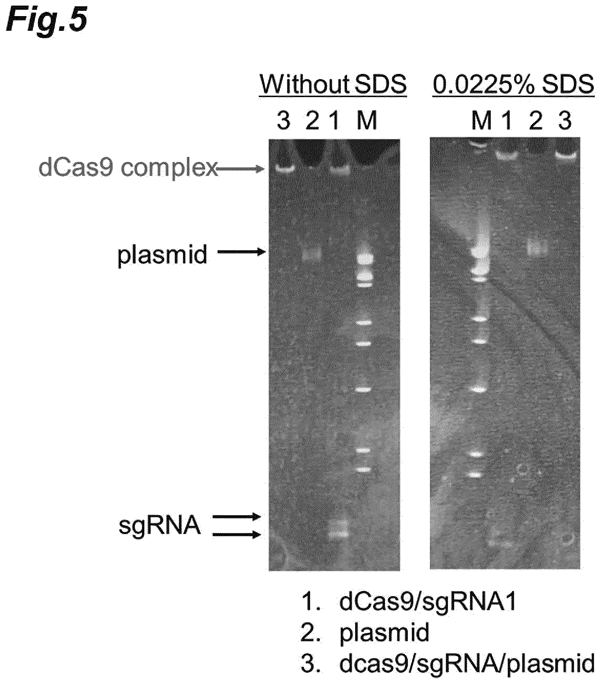

After electrophoresis, the stained gel photograph is shown in FIG. 5. Both in the presence and absence of SDS, the band of the plasmid alone observed in Lane 2 disappeared by adding dCas9/sgRNA, and the band was shifted upward in the gel (Lane 3), suggesting that about 0.0025% SDS does not affect the formation of the complex.

### (Complex Formation in Vitro)

A mixed solution of 6 µl of 0.15 µg/µl dCas9 and 3 µl of 2.3µM sgRNA, 2 µl of 10× H buffer, and 4.5 µl of MilliQ (trademark) water were added to 4.5 µl of a sample which was lysed in Channel 6, separated in Channel 1, and collected from the outlet (nucleic acid recovery port) of Channel 1, and the mixture was incubated at 37°C for 60 minutes. To 2.5 µl of the solution, 7.5 µl of a buffer (280 mM KCl, 20 mM DTT, 20 mM MOPS (pH 7)), 3.1 µl of MilliQ (trademark) water, and 1.9 µl of SLO (Streptolysin O, manufactured by BioAcademia) (final concentration: 1000 nM) were added, and the resulting mixture was used for evaluation in nanopores.

### (Evaluation of Current Inhibition by Nucleic Acid-Protein Complex Formed in Vitro)

4.7 µl of a buffer (140 mM KCl, 10 mM DTT, and 10mM MOPS (pH 7) was added to pores on a positive electrode side of a microdevice capable of reconstituting an artificial lipid bilayer membrane by a liquid contact method. 4.7 µl of the complex solution described above was added to a negative electrode side. Subsequently, lipid (DphPC(1,2-diphytanoyl-sn-glycero-3-phosphocholine) manufactured by Avanti Polar lipids: Cholesterol manufactured by Sigma = 1:1 (mol/mol)) was added to each pore, and this was set in PICO2 (manufactured by Tesla). Under a voltage of 100 mV, a lipid bilayer membrane (membrane capacity 65 to 70 pF) suitable for membrane protein insertion was formed using a trace rod. Pore formation due to association of membrane proteins on the lipid bilayer membrane and entry of molecules into the pore were detected by a change in current value. Analysis was performed by Clampex (free software), and blocking conductance calculated from the current value when the current in the pore was inhibited and the time (Duration time) when the current was inhibited were calculated.

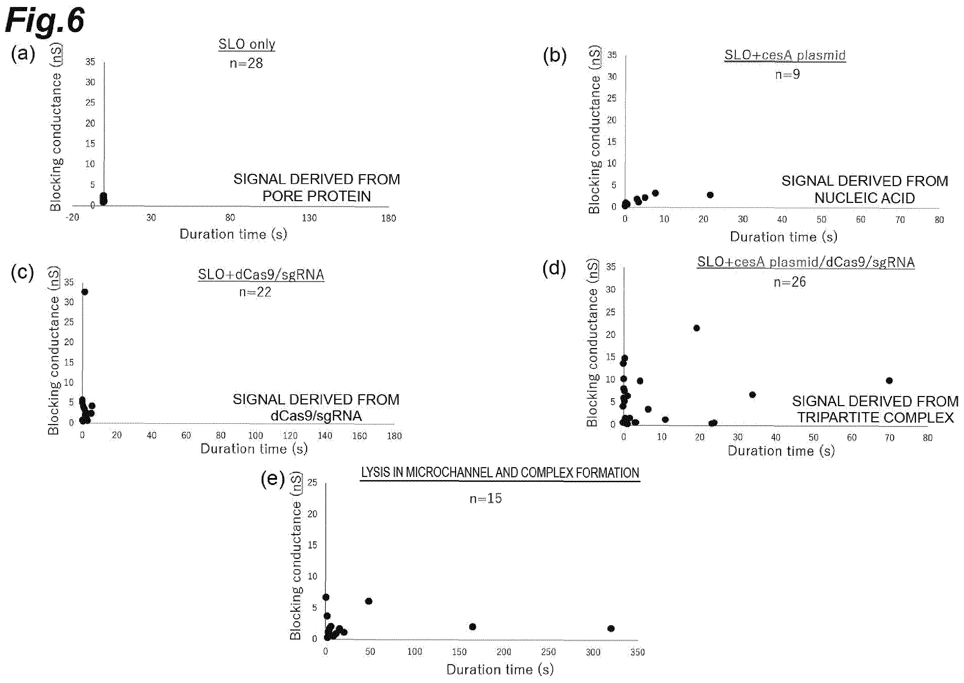

FIG. 6(D) illustrates a result of plotting the detected Blocking conductance and Duration time. A signal with a long Duration time, which was not observed when only the SLO (FIG. 6(A)), the SLO and the cesA plasmid (FIG. 6(B)), or the SLO and dCas9/sgRNA (FIG. 6(C)) were added, was detected only when the SLO and the lysate solution recovered from the microchannel outlet were added. From this result, it was suggested that a signal with a long Duration time was observed only when the complex of the dCas9/sgRNA and the target cesA plasmid entered the inside of the pore.

### (Formation of Complex in Microchannel)

The secondary channel outlet of Channel 1 and the inlet of Channel 6 were connected by a 1 mm diameter tube. From the other inlet, a solution in which 0.12 µg/ml dCas9, 2.7µM sgRNA, and 110× H buffer were mixed flowed in at 8.0 µl/min using a syringe pump. Samples were taken from the outlet and subjected to current inhibition evaluation experiments.

### (Evaluation of Current Inhibition by Nucleic Acid-Protein Complex Formed in Microchannel)

In the same manner as described above, the nucleic acid-protein complex formed in the microchannel was subjected to the evaluation of the current value by PICO2. The results are shown in FIG. 6(E). As in a case where the complex formed in vitro was evaluated (FIG. 6(D)), a signal with a long Duration time was detected. From this result, it was suggested that the complex formed in the channel entered the inside of the pore and was detected by a change in the current value.

### Example 2 Study of Channel Structure of Lysis Section (Comparison of Viability Rates by Channel Structure)

In addition to Channel 6 used in Example 1, a plurality of channels were designed so that the mixing of the bacteriolytic agent and the microorganism in the channel proceeded more efficiently, and among them, Channel A, C, D, G, H, I, and J channels were used for evaluation (FIG. 10). Details of the structure of these Channels are shown in FIGS. 32 to 35. The channel lengths (lengths from 2b to 2c) of Channel 6 and Channel A were both 2.5 cm, and the width (diameter) of the channel was 100 µm unless otherwise specified. 0.1% SDS and a microbial suspension (cell count concentration: 10¹⁰ cfu/ml) were each fed at 5.0 µl/min using a syringe pump, and a sample was collected from the outlet (2c). With respect to the colony count used in the method for evaluating the degree of lysis, the recovered liquid was diluted 10⁵ times with peptone water, and seeded on a 100 µl plate, and cultured overnight at 30°C. The number of bacteria before flowing into the channel was compared with the number of bacteria counted in the recovered liquid, and the viability rate was calculated.

### (Test Result: Comparison of Viability Rates by Channel Structure)

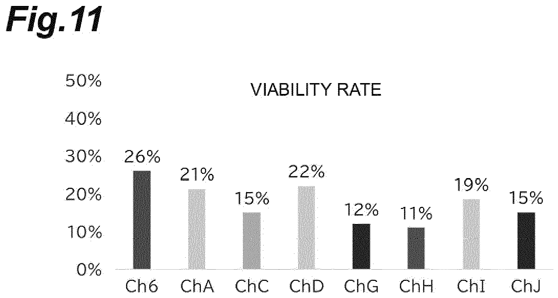

For Channel G and Channel J, clogging was confirmed in the channel. As compared with Channel 6 of (a), the viable cell counts decreased in all the channels, suggesting that lysis efficiently proceeded (FIG. 11). In particular, the most remarkable effect was observed in Channel H, and then the effect was observed in the order of Channel C, Channel I, Channel A, and Channel D.

### Example 3 Study of Bacteriolytic Agents

### (Comparison of Viability Rates by Bacteriolytic Agent)

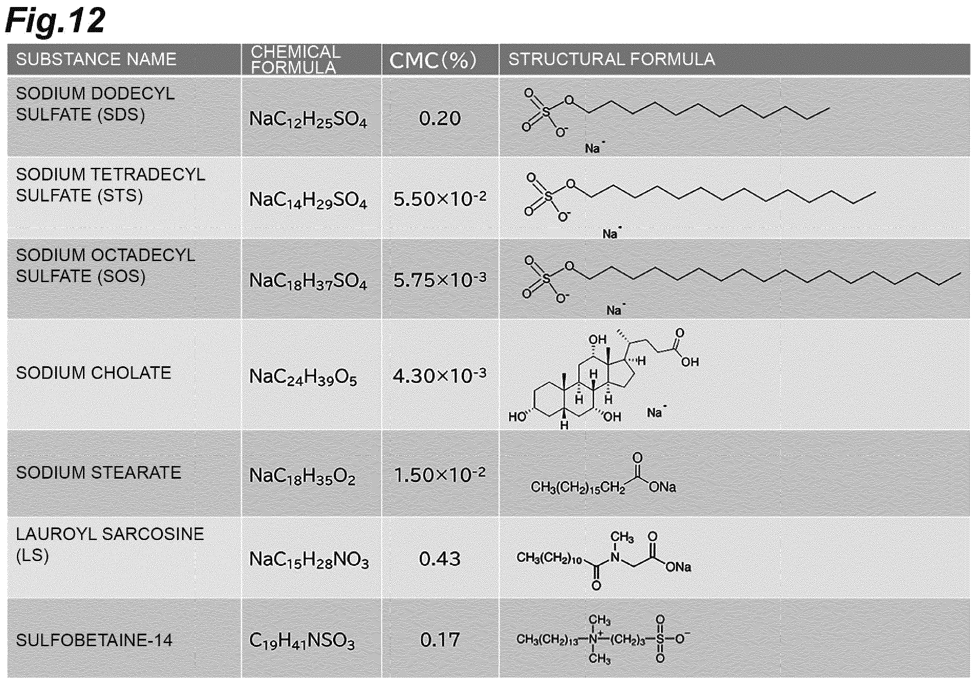

The degrees of lysis of various kinds of bacteriolytic agents were compared. Channel 6 of Example 1 was consistently used for evaluation. The bacteriolytic agent used for the evaluation is shown in FIG. 12. Various bacteriolytic agents and a microbial suspension (cell count concentration: 10⁹ cfu/ml) were each fed at 5.0 µl/min using a syringe pump, and a sample was collected from the outlet (2c). In addition, the addition concentrations were all prepared so that the final concentration was the critical micelle concentration (CMC). With respect to the colony count used in the method for evaluating the degree of lysis, the recovered liquid was diluted 10⁴ times with peptone water, and seeded on a 100 µl plate, and cultured overnight at 30°C. The number of bacteria before flowing into the channel was compared with the number of bacteria counted in the recovered liquid, and the viability rate was calculated.

### (Test Result: Comparison of Viability Rates by Bacteriolytic Agent)

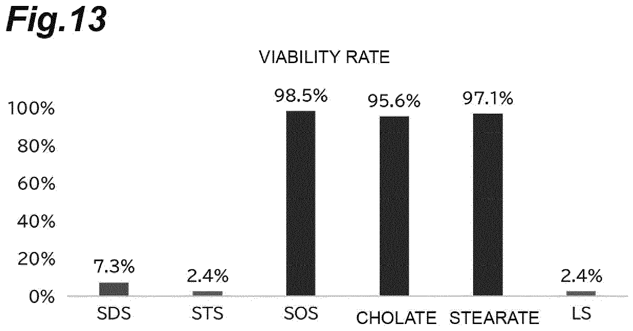

With respect to SDS, a higher lytic effect was observed in sodium tetradecyl sulfate (STS) and lauroyl sarcosine (LS) (FIG. 13). On the other hand, in sodium octadecyl sulfate (SOS), sodium cholate, and sodium stearate, the viability rate was high, and the lytic effect was hardly exhibited. From this result, it was considered that STS and LS are effective as the bacteriolytic agent.

### (Comparison of Degrees of Lysis between BugBuster and Sulfobetaine)

Upon receiving the result of the comparison of the viability rates, the degrees of lysis were compared in more detail by comparing the nucleic acid recovery rate by quantitative PCR. As primers, enzymes, and the like, those attached to the CycleavePCR^{™} Bacillus cereus (CRS gene) Detection Kit (Takara Bio Inc.) were used. The recovered liquid was purified by QIAquick Gel Extraction Kit (manufactured by Qiagen) and verified under the following conditions. Hold (initial denaturation): 95°C, 10 s → 3 step PCR: 45 cycles (95°C, 5 s → 55°C, 10 s → 72°C, 20 seconds). A Ct value of the sample that was left standing overnight after addition of the bacteriolytic agent to a bacterial fluid at the same concentration in vitro was regarded as a "100% lysed" control, and the lysis rate of the sample lysed in the channel with each bacteriolytic agent was evaluated. Prior to the evaluation of STS and LS considered to be effective, a commercially available lysis reagent BugBuster (trademark) Protein Extraction Reagent (Merck), which is considered to exhibit a high lytic effect, was used for verification. Sulfobetaine-8 (SB-8) and sulfobetaine-14 (SB-14) in which a carbon chain of the sulfobetaine was modified were added to the comparative subjects, and the nucleic acid recovery rates were compared.

### (Test Result: Comparison of Degrees of Lysis between BugBuster and Sulfobetaine)

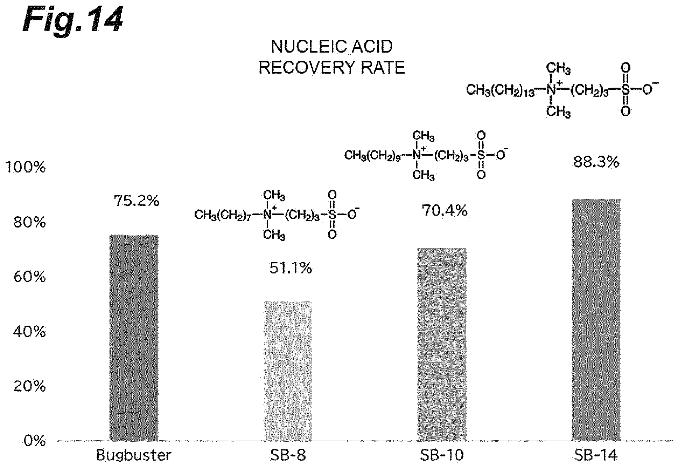

The results are shown in FIG. 14. Comparing the recovery amounts of various sulfobetaines, the recovery rate of SB-8 having a shorter carbon chain was lowered, and the recovery rate of SB-14 having a longer carbon chain was improved. From this result, SB-14 was also determined to be effective as a bacteriolytic agent similarly to STS and LS, and was used for the next verification.

### (Comparison of Degrees of Lysis in STS, LS, BugBuster, and SB-14)

For STS, LS, BugBuster, and SB-14 for which a high lytic effect was observed in previous studies, the nucleic acid recovery rates including SDS were compared in series.

### (Test Result: Comparison of Degrees of Lysis in STS, LS, BugBuster, and SB-14)

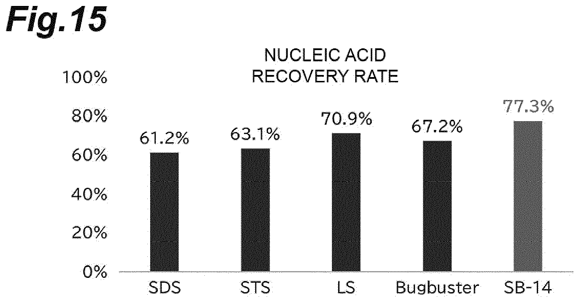

With respect to SDS, a higher nucleic acid recovery rate was observed with any of the bacteriolytic agents, but among them, SB-14 showed the highest recovery rate (FIG. 15). From this result, it was determined that SB-14 was optimal as a bacteriolytic agent.

### Example 4 Study of Combination of Lysis Section Channel Structure and Bacteriolytic Agent

### (Comparison of Degrees of Lysis in Case of Combination of Bacteriolytic Agent and Channel Structure)

Regarding the lytic effect, whether a synergistic effect between the channel structure and the bacteriolytic agent was observed or not was verified by comparing the amount of nucleic acid recovered. Channel 6 and Channel H of Example 1 were used as a channel structure, and SDS, BugBuster, and SB-14 were used as a bacteriolytic agent.

### (Test Result: Comparison of Degrees of Lysis in Case of Combination of Bacteriolytic Agent and Channel Structure)

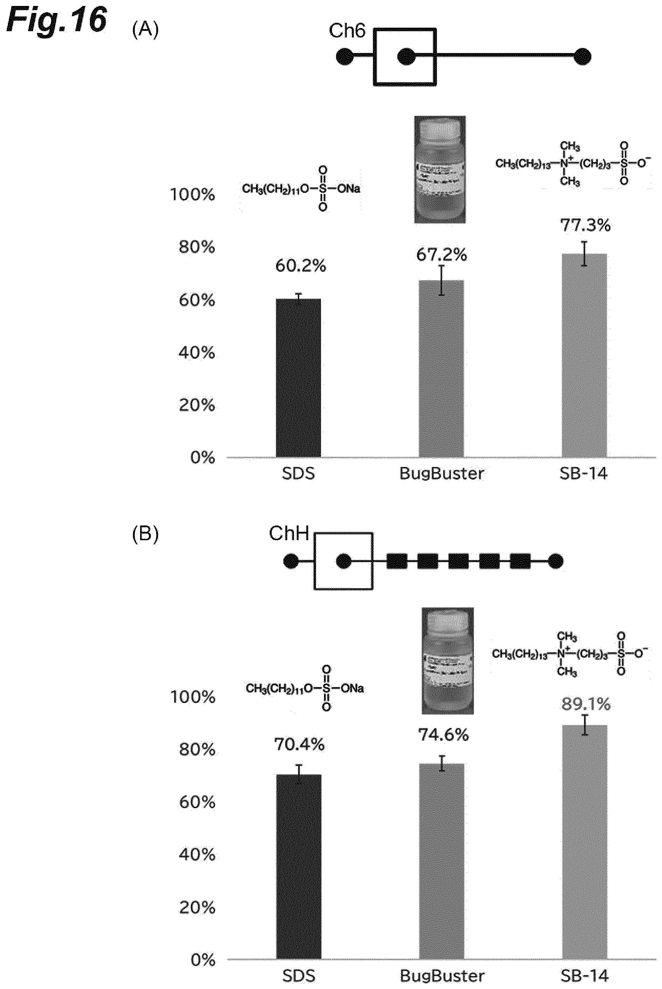

When comparison was performed in the channel of Channel 6, regarding the bacteriolytic agent, the lysis rate was higher in the order of SB-14, BugBuster, and SDS (FIG. 16(A)). This tendency was similar in Channel H (FIG. 16(B)). In addition, when the comparison was performed using the channel structure, Channel H exhibited a higher lytic effect than Channel 6 in any of the bacteriolytic agents. From the results so far, it was suggested that "Channel H" is appropriate as a channel having a high lytic effect, and "SB-14" is appropriate as a bacteriolytic agent, and by combining both, the lysis rate is improved from 60% to 89% as compared with the case of using SDS and Channel 6.

### Example 5 Study of Other Food Poisoning Bacteria

### (Verification of Lysis of Other Food Poisoning Bacteria)

It was verified whether the present lysis step could be applied to other food poisoning bacteria. Regarding the channel, Channel H was used, and three kinds of SDS, generally used benzalkonium chloride (BC), and SB-14 were used as the bacteriolytic agent. As food poisoning bacteria of a subject, Listeria monocytogenes, Staphylococcus aureus, and Salmonella enterica were used. As each inoculum, CycleavePCR^{™} Listeria monocytogenes (inlA gene) Detection Kit, CycleavePCR^{™} Staphylococcus aureus (DnaJ gene) Detection Kit, and CycleavePCR^{™} Salmonella Detection Kit Ver. 2.0 which were quantitative PCR kits manufactured by Takara Bio Inc. were used. Pretreatment and measurement conditions were the same as for the Bacillus cereus.

### (Test Result: Verification of Lysis of Other Food Poisoning Bacteria)

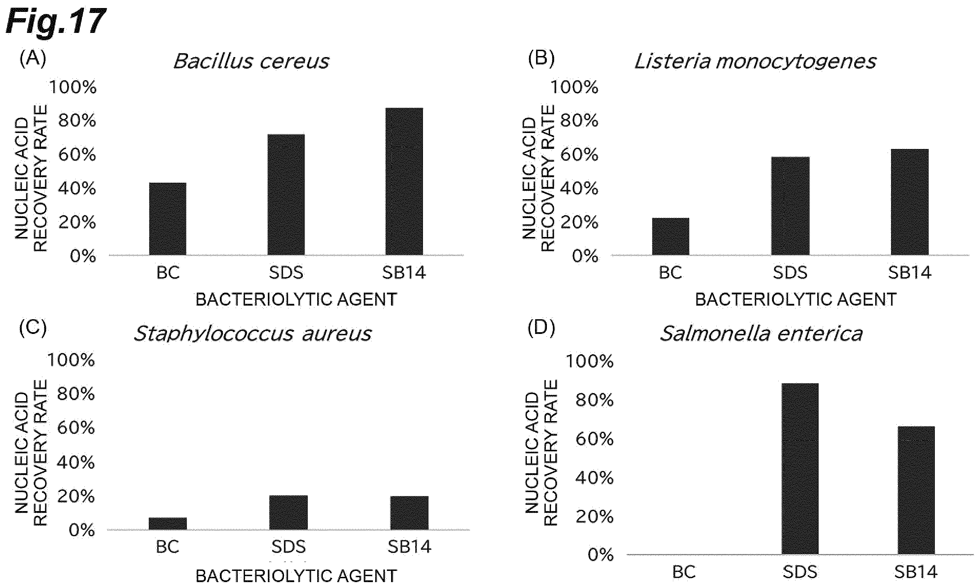

The results are shown in FIG. 17. It was suggested that all of the four bacterial species were lysed in the channel. For each strain, Listeria monocytogens showed a similar tendency to Bacillus cereus (FIGS. 17(A) and 17(B)). On the other hand, in Staphylococcus aureus, SDS and SB-14 showed almost the same lysis rate (FIG. 17(C)), and in Salmonella enterica, SDS showed a higher lysis rate (FIG. 17(D)). This is considered to be due to a difference in membrane structure (Gram-positive/negative). From this result, it was suggested that the present lysis step can be applied to various food poisoning bacteria by setting an appropriate bacteriolytic agent according to the bacterial species.

### Example 6 Study of Complex Formation

### (Verification of Complex Forming Step Using FRET Evaluation System)

As a detection system for evaluating complex formation, fluorescence resonance energy transfer (FRET) was used. The FRET method refers to a phenomenon in which excitation energy is directly transferred between two dye molecules close to each other. The energy of the light absorbed by one molecule moves to the other, and fluorescence is emitted from the molecule that has received the energy.

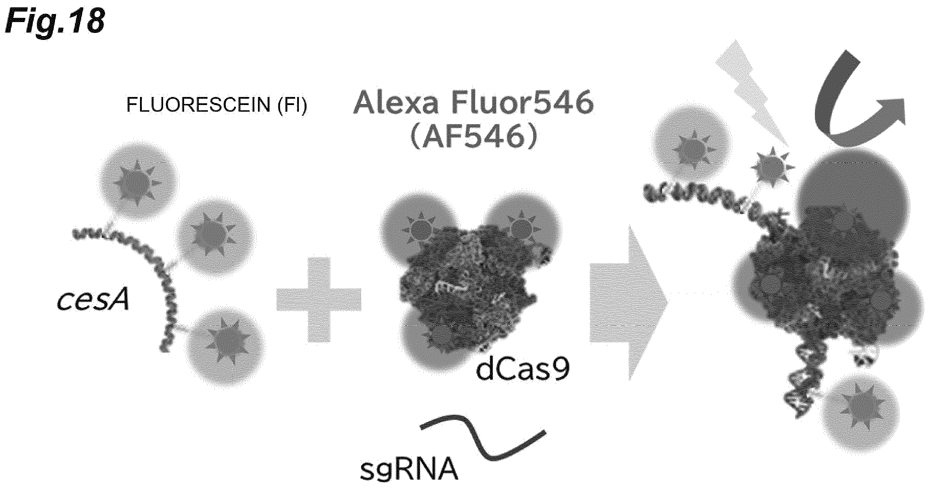

Using this principle, the complex formation of the cesA plasmid, which is a nucleic acid derived from Bacillus cereus, and the protein dCas9 was evaluated. Specifically, when cesA and dCas9 are modified with different fluorescent dyes and both of them form a complex, the modified green and red fluorescent dyes come close to each other to generate FRET. A conceptual diagram is shown in FIG. 18. Complex formation was quantitatively evaluated using the red fluorescence intensity increased by FRET as an index.

### (Fluorescent Dye Modification to cesA and dCas9)

For each of cesA and dCas9, dye modification was performed using a commercially available kit. Fluorescein was modified for cesA, and Alexa Fluor546 was modified for dCas9. First, regarding the cesA, Label IT (trademark) Tracker^{™} Intracellular Nucleic Acid Localization Kit (Takara Bio Inc.) was used. 37.5 µl of MilliQ (trademark) water, 5 µl of 10× Labeling Buffer, 5 µl of 1 mg/ml cesA plasmid, and 1 µl of Label IT Reagnent were mixed and incubated at 37°C for 1 hour for reaction. After the reaction, purification was performed by ethanol precipitation to obtain a desired product.

For dye modification to dCas9, Alexa Fluor^{™} 546 NHS Ester (Succinimidyl Ester, Invitrogen) was used. 1 µl of 10 mg/ml Alexa Fluor 546, 6.7 µl of 15 mg/ml dCas9, and 13.3 µl of 0.2M NaHCO₃ (pH 8.3) dissolved in DMF were mixed, and the mixture was incubated at room temperature for 1 hour for reaction. Purification was performed using an Amicon Ultra 0.5 mL centrifugal filter (Merck) to obtain a desired product.

### Example 7 Study of Various Channel Structures in Complex Forming Section

### (Comparison of Complex Formation Reaction by Various Channel Structures)

The channel structure shown in FIG. 19 was used for evaluation of the complex forming ability. Channel 7 was designed to have a channel length twice that of Channel 6, and Channel 5 was designed to have a channel length half that of Channel 6. In addition, the Channel I was applied to design the Channel I4 in which the number of branches and confluence parts was doubled. Since it was considered that there was a concern of clogging in the channel due to an increase in the number of branches and confluence parts, the channel width was designed to be twice as large from the middle. Specifically, the channel width from 4b to the midpoint between 4b and 4c (channel central portion 4d) (4b to 4d, length 1.25 cm) was 100 µm, and the channel width from the midpoint between 4b and 4c (channel central portion 4d) to 4c (4d to 4c, length 1.25 cm) was 100 µm to 200 µm. In order to see the influence of the change in the channel width, Channel I2 in which the number of branches and confluence parts were equal to that of Channel I and the channel width was changed twice from the middle was also used for evaluation. For various channels, fluorescein-modified cesA and Alexa Fluor546 modified dCas9 were added to 1× H Buffer (manufactured by Takara) so as to have final concentrations of 0.25 µg/ml and 2.5 µg/ml, respectively. In order to prevent dye adsorption in the channel, BSA was added so as to have a final concentration of 0.2%. The cesA solution and the dCas9 solution were separately fed from each of the two inlets (4a and 4b) at 5.0 µl/min using a syringe pump, and 10 µl of each sample was collected from the outlet 4c. In advance, 90 µl of 1× H Buffer was added to Nunc^{™} MicroWell^{™} 96 Well, Nunclon Delta-Treated, and Flat-Bottom Microplate (Thermo Fisher Scientific Inc.), and 10 µl of a sampling liquid was added to the well so that a total of 100 µl was obtained. After completion of the collection, fluorescence spectra of various samples were measured. For the fluorescence measurement, a fluorescence plate reader Infinite (trademark) M200 (TECAN) was used. The excitation wavelength was set to 430 nm, the fluorescence intensity was measured every 1 nm, and a value obtained by averaging the fluorescence intensities of 572 nm to 581 nm was used. The sample obtained by adding the cesA solution and the dCas9 solution in the microtube and incubating for 1 hour was regarded as a "100% complex formed" control, and the complex formation rate of a sample in which the complex was formed in the channel was evaluated in comparison with the fluorescence intensity of the sample solution.

### (Test Result: Comparison of Complex Formation Reactions by Various Channel Structures)

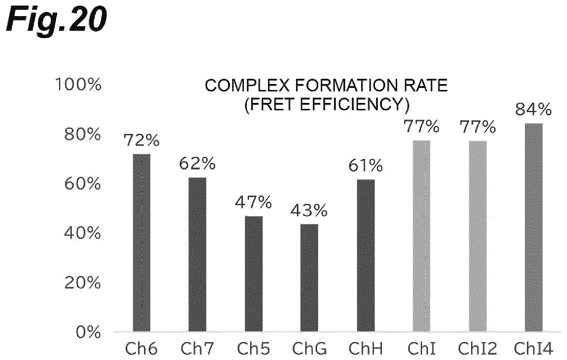

FIG. 20 shows the results of comparing the complex formation rates in various channels. In the Channel 5, the Channel 7, and the Channel G in which the channel length was changed from the Channel 6 of Example 1, no improvement in the formation rate was observed. Furthermore, even in Channel H in which a high lytic effect was observed in the lysis step, no remarkable improvement was observed. On the other hand, in Channel I provided with a branch and a confluence part, the complex formation rate was improved. The intermediate change of the channel width did not affect the complex formation, and clogging in the channel was not actually observed. Further, in Channel I4 in which the number of branches and confluence parts was increased, the complex formation rate was further improved. From this result, it was suggested that the "branch and confluence" structure in the channel affected the complex formation, and among the studied Channels, Channel I4 was optimal.

### Example 8 Study of Integrated Detection Device

### (Evaluation of Degrees of lysis in Pretreatment Integrated Channel)

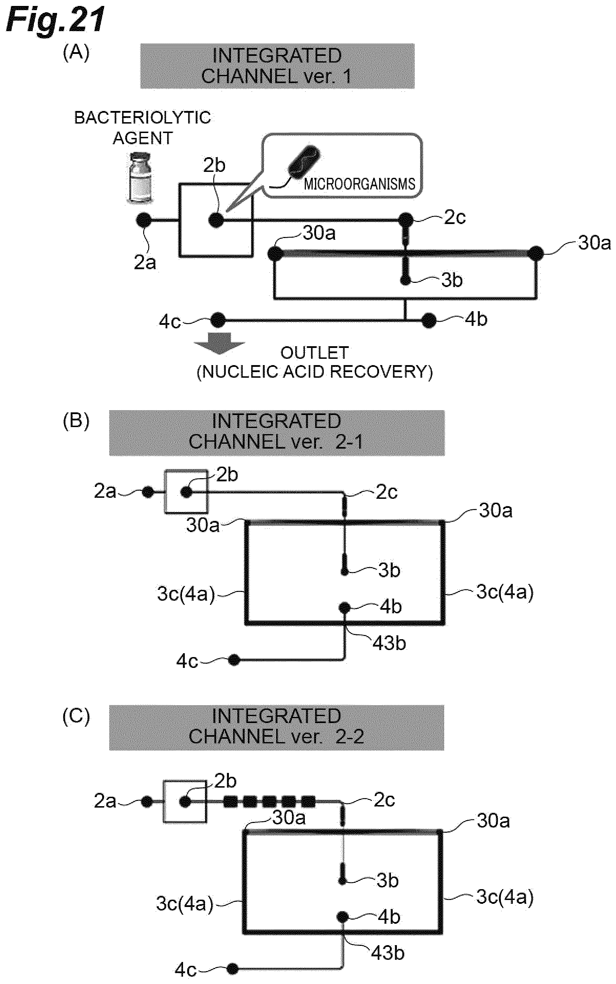

In the present example, the effects of the device in which the "lysis section", the "separation section", and the "complex forming section" are integrally connected (which may be referred to as a "pretreatment integrated channel" in the present specification) were studied. In the same manner as in Example 1, a pretreatment integrated channel was prepared, and whether or not nucleic acid could be recovered from the outlet 4c, which is the outlet of the integrated channel, was verified by adding the microbial solution and the bacteriolytic agent from the inlet 1 (2a) and the inlet 2 (2b), respectively. Three types of integrated channels were used for evaluating the degree of lysis. The first studied integrated channel ver. 1 (FIG. 21(A)) was coupled without considering the resistance in the channel. A channel whose channel structure is appropriately designed in view of the resistance in the channel is ver. 2-1 (FIG. 21(B)). Further, a channel incorporating the structure of Channel H exhibiting a high lytic effect is ver. 2-2 (FIG. 21(C)). The detailed structure of ver. 2-1 is shown in FIGS. 22 and 23.

### (Test Result: Evaluation of Degrees of lysis in Pretreatment Integrated Channel)

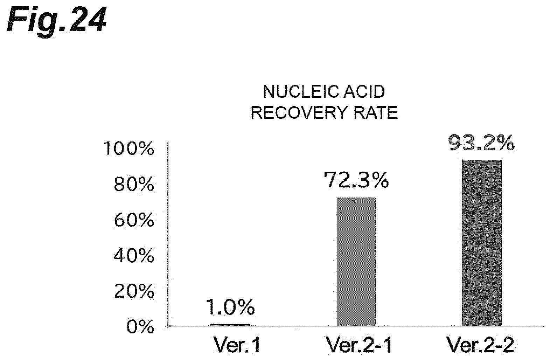

In ver. 1, the amount of nucleic acid recovered from the outlet 4c remained at 1% (FIG. 24). As a cause of this, it was considered to be the possibility that since the design was made without considering the resistance in the channel, backflow occurred in the channel, and most of the nucleic acid did not reach the outlet 4c. On the other hand, in ver. 2-1 in which the channel structure was appropriately designed in view of the resistance in the channel, it was possible to recover nearly 70% of the whole nucleic acid. Furthermore, in ver. 2-2, due to high lytic effect thereof, the recovery rate increased to about 90% (FIG. 24).

### (Verification of Complex Formation in Pretreatment Integrated Channel)

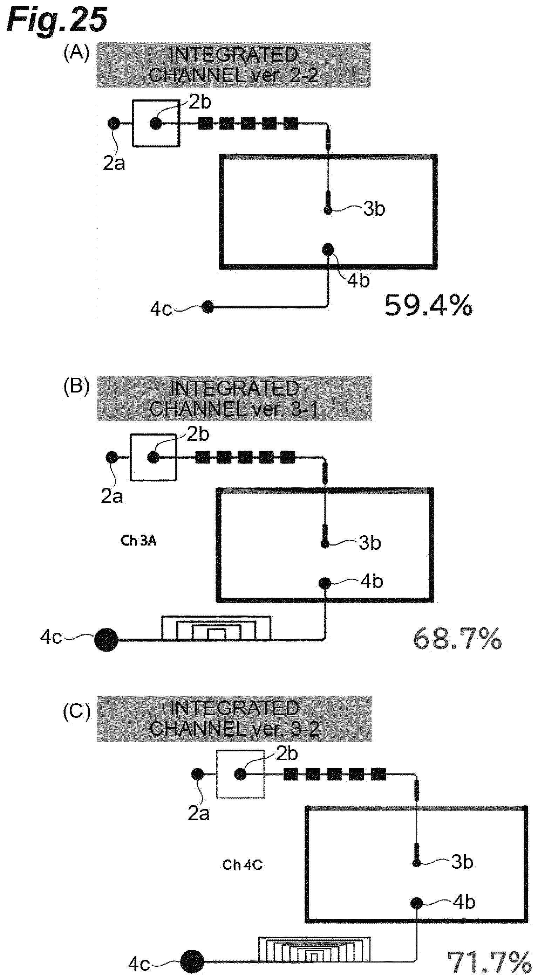

Subsequently, the complex formation rate in the pretreatment integrated channel was also studied. The channel structure in which the complex forming ability was improved by the channel structure modification was incorporated into the integrated channel. FIG. 25 shows a structure of a newly designed pretreatment integrated channel. For ver. 2-2 (FIG. 25(A)) in which the highest lytic effect was observed, in the complex forming section, the channel incorporating Channel I was set as ver. 3-1 (FIG. 25(B)), and the channel incorporating Channel I4 was set as ver. 3-2 (FIG. 25(C)). The complex formation rates were compared among these three kinds of pretreatment integrated channels.

### (Test Result: Verification of Complex Formation in Pretreatment Integrated Channel)

The flow complex forming abilities were compared in ver. 2-2, ver. 3-1, and ver. 3-2, and the highest complex formation rate was confirmed in ver. 3-2 (FIG. 25). From this result, it was found that the same tendency as in the comparative verification of the complex formation rate in the single step was observed. From the above results, it was suggested that the same tendency was observed in the integrated channel by using the channel structure in which the effect was observed in various steps.

### (Production of Pretreatment and Detection Integrated Channel)

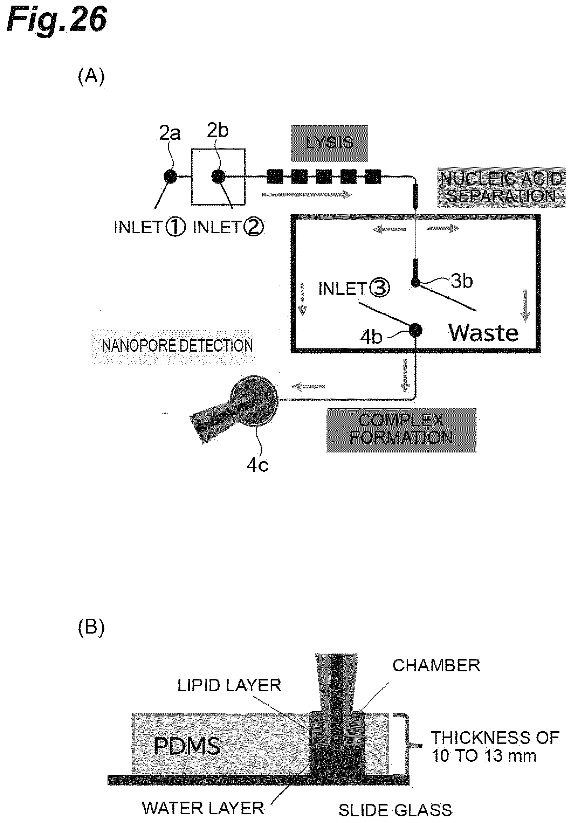

By regarding the outlet 4c of the pretreatment integrated channel as a detection chamber (detection section), an integrated device (may be referred to herein as a "pretreatment and detection integrated channel" or simply as an "integrated channel") that can seamlessly perform pretreatment to detection was prepared. A hole having a diameter of 6 mm was made in the outlet 4c of the above-described pretreatment integrated channel ver. 2-2 so that a certain amount of solution was accumulated in the detection section (FIG. 26(A)). In addition, in consideration of handling of detection, PDMS as a material was adjusted to a thickness of 10 to 13 mm (FIG. 26(B)). By adopting such a structure, it is possible to add a lipid layer on an aqueous layer which is a recovery liquid containing a complex and to install a glass silver electrode which is a detection section near an interface between both layers.

### (Electrochemical Detection of Bacillus Cereus Using Integrated Device)

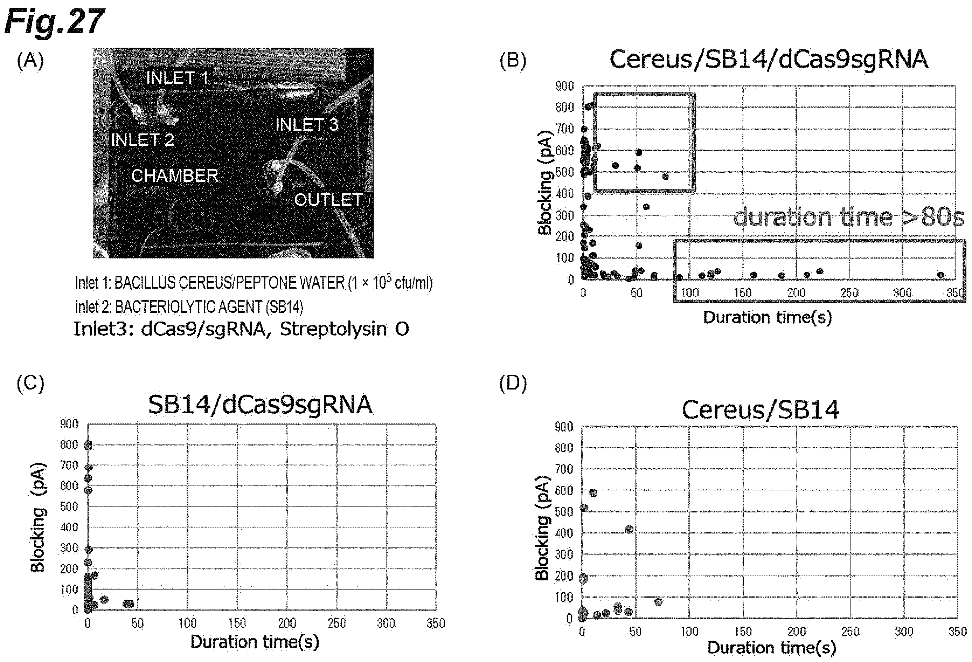

In the integrated device (FIG. 27(A)), Bacillus cereus (1 × 10³ cfu/ml) from the inlet 1, a bacteriolytic agent (sulfobetaine-14) from the inlet 2, and a mixed solution of the dCas9/sgRNA complex and Streptolysin O (final concentration: 30 nM) dissolved in 1× H buffer (manufactured by TAKARA) from the inlet 3 were caused to flow into the device at flow rates of 5.0 µl/min, 5.0 µl/min, and 4.0 µl/min, respectively. After 42 minutes, the solution in the chamber was removed, and then the inflow was started again. After about 15 minutes, the syringe pump was stopped, and DPhPC/Cholesterol (= 1:1 (mol/mol)) dissolved in hexadecane was added. The silver wire glass electrode was inserted into an oil layer, and a reference electrode was inserted into a water tank into which the reference electrode flowed in, and a voltage was applied at 100 mV. A lipid bilayer membrane was formed by moving the electrode up and down in a Z-axis direction with a hydraulic manipulator (Shoji, K., et al., Anal. Chem., 2020, 92, 10856-10862), and a stepwise increase in the current value due to pore insertion and a decrease in the current value when the molecule was inserted into the pore were detected. After conversion into an ABF file, the file was analyzed by Clampex (free software).

### (Test Result: Detection of Bacillus Cereus Using Integrated Device)

FIGS. 27(B), 27(C), and 27(D) show the results of analyzing and plotting each signal, with a current value dropped from a current value observed in a state where the pore is opened as Blocking (pA) and a time during which the current value is maintained as Duration time. It has been revealed that, in a case where Bacillus cereus, a bacteriolytic agent, and dCas9/sgRNA flowed into the channel (FIG. 27B), there were some signals that were not observed in a case where the bacteriolytic agent and dCas9/sgRNA were flowed in (FIG. 27C) or in a case where Bacillus cereus and a bacteriolytic agent flowed in (FIG. 27D). First, a signal having 50 pA or less at a Duration time of 100 seconds or more was observed (FIG. 27(B)). Next, a signal having a Duration time of about 0 to 70 seconds at 400 pA or more was observed (FIG. 27(B)).

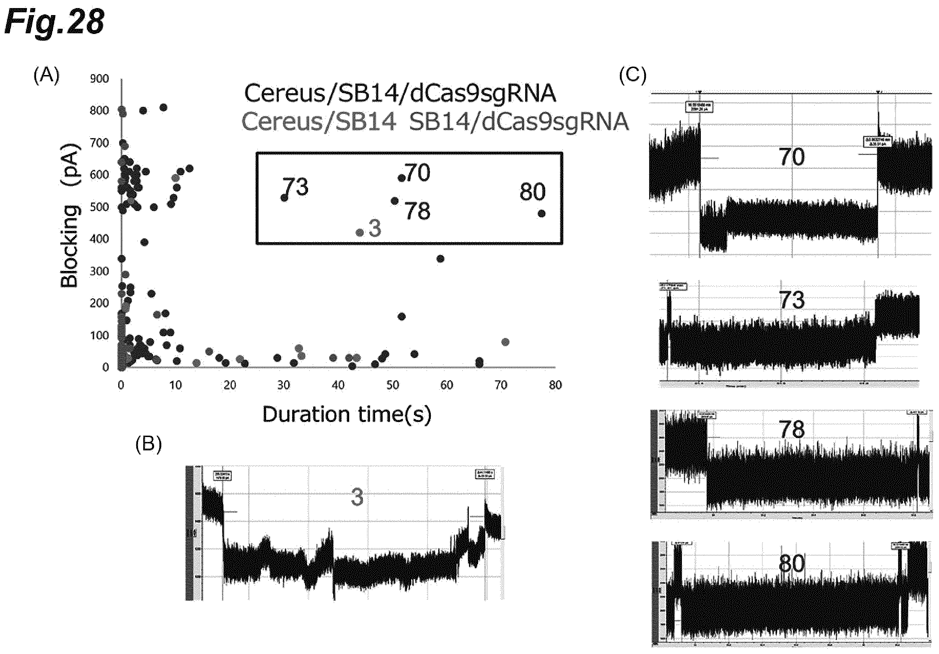

FIG. 28(A) illustrates five signals with Blocking of 400 pA or more and a Duration time of 30 seconds or more. As for the case where Bacillus cereus, a bacteriolytic agent, and dCas9/sgRNA flowed into the channel (Signal No. 70, 73, 78, and 80), when examining the shape of the signals, a flat signal was observed as in the baseline (FIG. 28(C)) when the molecule entered the pore and the current value decreased. On the other hand, in a case where Bacillus cereus and a bacteriolytic agent flowed in (signal number 3), signal disturbance was observed after the current value dropped (FIG. 28(B)).

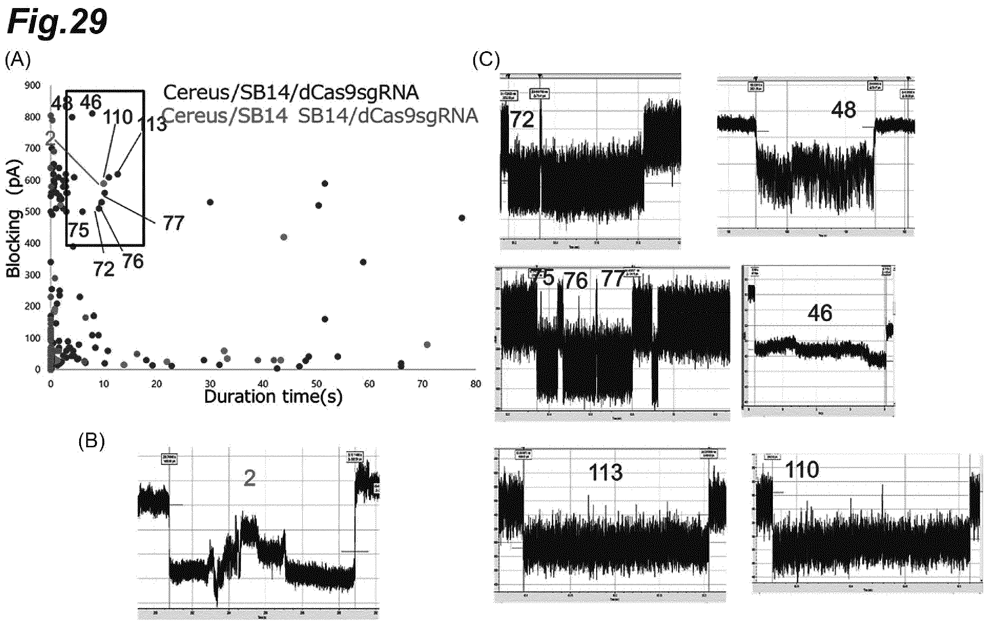

Similarly, also for 9 signals (FIG. 29(A)) having Blocking of 400 pA or more and a Duration time of 4 seconds or more and 20 seconds or less, a flat signal (FIG. 29(C)) was observed when Bacillus cereus, a bacteriolytic agent, and dCas9/sgRNA flowed in, and signal disturbance was observed when Bacillus cereus and a bacteriolytic agent flowed in (FIG. 29(B)).

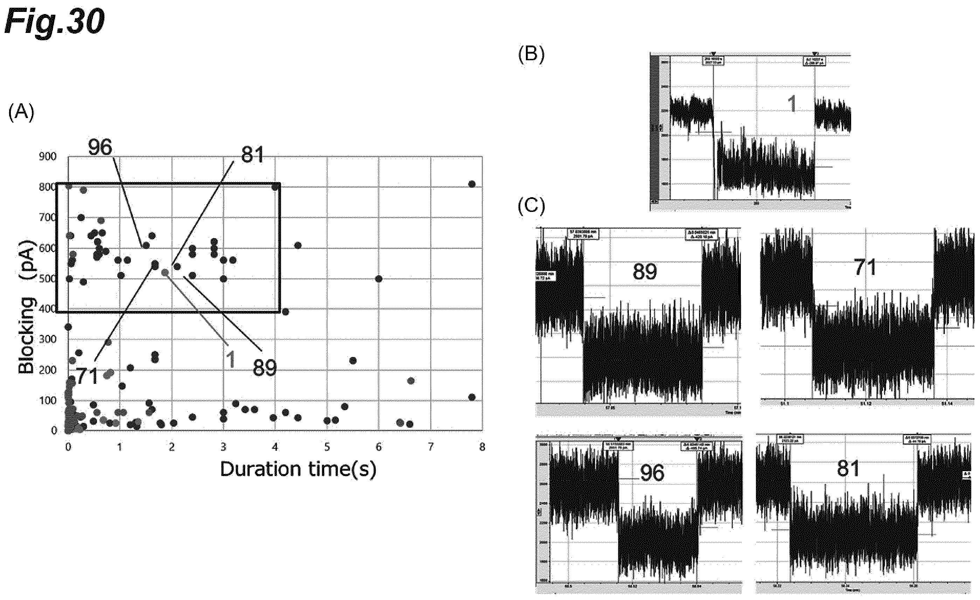

On the other hand, with respect to a signal having Blocking of 400 pA or more and a Duration time of 1 second or more and 4 seconds or less (FIG. 30(A)), the same flat signal was observed even when Bacillus cereus, a bacteriolytic agent, or dCas9/sgRNA flowed in or when Bacillus cereus and a bacteriolytic agent flowed in (FIGS. 30(B) and 30(C)). It is considered that when a bacteriolytic agent and dCas9/sgRNA flowed in, there are many signals with a Duration time of 1 second or less (FIG. 27(C)), and thus the pore is hardly clogged by dCas9/sgRNA alone. On the other hand, it is considered that in a case where Bacillus cereus and a bacteriolytic agent (SB14) flowed in, since there are many signals with a Duration time of 1 second or more, it is probably negatively charged, and thus the nucleic acid derived from Bacillus cereus is likely to be clogged in the pore (FIG. 27(D)).

From the above results, it is considered that in a case where the nucleic acid derived from Bacillus cereus is clogged in the pore, the nucleic acid moves inside the pore, and thus the signal is disturbed. On the other hand, it is considered that since the nucleic acid derived from Bacillus cereus/dCas9/sgRNA complex fits the pore diameter and volume, the movement inside the pore is limited, and as a result, a flat signal is obtained. On the other hand, there was no clear difference between the signals with blocking of 400 pA or more and a Duration time of 4 seconds or less. This result indicates that the behavior of the molecule inside the pore does not appear as a signal unless the molecule is clogged in the pore for a certain period of time. However, although the difference is currently unknown visually, if the difference between these signals can be distinguished in the future by Fourier transform, machine learning, or the like, the sensitivity can be expected to be improved.

### Example 9 Detection of Bacillus Cereus by Integrated Device

### (Collection and Lysis of Bacillus Cereus in Adjusted Milk Powder)

1 bag (13 g) of a stick of adjusted milk powder (manufactured by Morinaga Milk Products Co., Ltd.) was dissolved in 100 ml of hot water and then cooled, and Bacillus cereus was added so as to have a final concentration of 1 × 10³ cfu/ml. Then, this was filtered through a cellose acetate membrane (manufactured by Advantec) having a pore size of 0.8 µm. The membrane was washed 3 times with 10 ml of saline. Finally, 0.5 ml of sulfobetaine-14 was loaded on the membrane, and then the lysate solution was recovered.

### (Electrochemical Detection of Bacillus Cereus in Adjusted Milk Powder Using Integrated Device)

The Bacillus cereus lysate solution recovered from the membranes from the inlet 1 and the inlet 2 of the integrated device (FIG. 27(A)) was allowed to flow into the device at a flow rate of 8.0 µl/min. After 20 minutes, the flow rate was changed to 5.0 µl/min, and at the same time, a mixed solution of the dCas9/sgRNA complex dissolved in 1× H buffer (manufactured by TAKARA) and Streptolysin O (final concentration: 30nM) was caused to flow from the inlet 3 at a flow rate of 4.0 µl/min. After 60 minutes, the solution in the chamber was removed, and then the inflow was started again. After about 15 minutes, the syringe pump was stopped, and DPhPC/Cholesterol (= 1:1 (mol/mol)) dissolved in hexadecane was added. The silver wire glass electrode was inserted into an oil layer, and a reference electrode was inserted into a water tank into which the reference electrode flowed in, and a voltage was applied at 100 mV. A lipid bilayer membrane was formed by moving the electrode up and down in a Z-axis direction with a hydraulic manipulator (Shoji, K., et al., Anal. Chem., 2020, 92, 10856-10862), and a stepwise increase in the current value due to pore insertion and a decrease in the current value when the molecule was inserted into the pore were detected. After conversion into an ABF file, the file was analyzed by Clampex (free software).

### (Test Result: Detection of Bacillus Cereus in Adjusted Milk Powder Using Integrated Device)

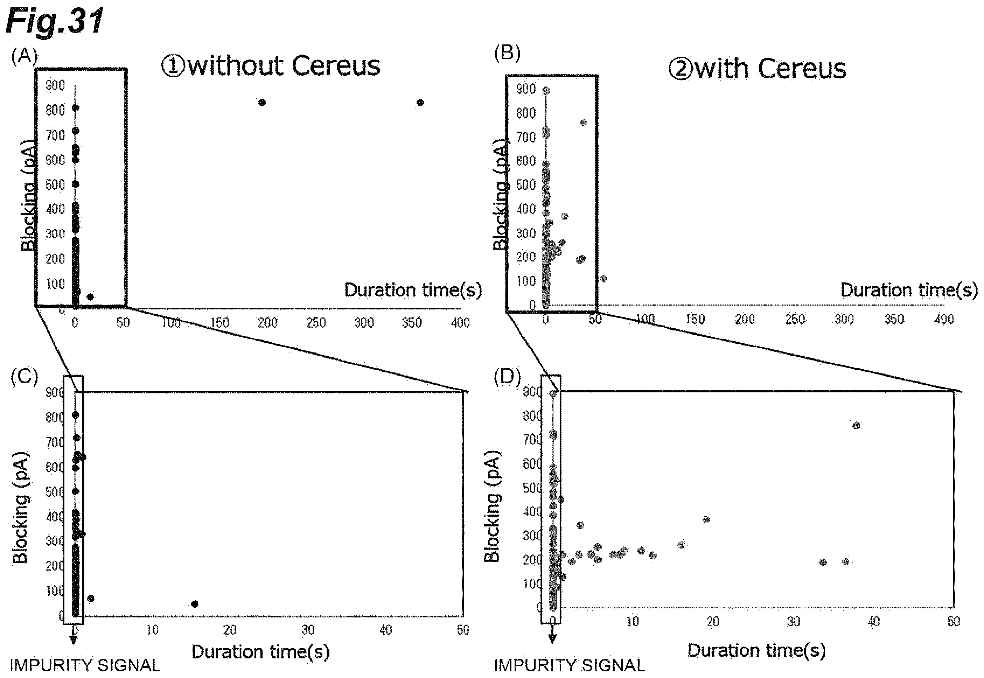

FIG. 31 shows the results of analyzing and plotting each signal as Blocking (pA) and a time during which the current value is maintained as Duration time. In a case where Bacillus cereus was not added, most of the signals had a Duration time of 1 second or less (FIGS. 31(A) and 31(C)). From this result, it is considered that impurities such as milk fat, protein, and lactose contained in the adjusted milk powder cannot enter the inside of the pore probably due to having no charge.

On the other hand, in a case where Bacillus cereus was added, in addition to a signal with a Duration time of 1 second or less, which is considered to be impurities, a signal not observed when Bacillus cereus was not added (signal with Blocking time of about 200 pA and Duration time of several seconds to several tens of seconds) was observed (FIGS. 31(B) and 31(D)). This result suggests that these signals are signals derived from Bacillus cereus. It has been reported that a quantitative PCR method known as a detection method targeting a gene of a microorganism is often false-negative due to inhibition of an enzyme reaction, inhibition of probe fluorescence, and inhibition of annealing of a primer by impurities in food (https://foodmicrob.com/pcr-detection-bacteria-from-food/). In the gene detection method using the nanopore described in the present application, impurities hardly enter the inside of the pore, and thus there is a possibility that only the target gene and the dCas9/sgRNA complex can be efficiently detected.

### Reference Signs List

- 1: Microfluidic device

- 2: Lysis section
- 2a: Sample receiving inlet
- 2b: Bacteriolytic agent receiving inlet
- 2c: Lysate solution discharging outlet
- 2S: Lysis channel
- 21: First lysis channel portion
- 22: Second lysis channel portion
- 23: Lysis branch channel portion
- 24: Lysis mixing channel portion
- 23a: Lysis branch part
- 23b: Lysis confluence part
- 3: Separation section
- 3a: Inlet
- 3b: Impurity recovery port
- 3c: Outlet
- 30: Separation unit
- 30a: First part that outputs first result
- 30b: Second part that outputs first result
- 31: First separation channel portion
- 311: First small diameter channel part
- 312: Large diameter channel part
- 313: Second small diameter channel part
- 32: Second separation channel portion
- 321: Small diameter channel part
- 322: First large diameter channel part
- 323: Second large diameter channel part
- 33: Separation branch channel portion
- 4: Complex forming section
- 4a: First result receiving inlet
- 4b: Complex forming substance receiving inlet
- 4c: Outlet for discharging complex
- 4d: Channel central portion
- 4S: Complex channel
- 41: First complex channel portion
- 42: Second complex channel portion
- 43: Complex branch channel portion
- 43a: Complex branch part
- 43b: Complex confluence part
- 44: Complex mixing channel portion
- 5: Detection section

## Claims

1. A microfluidic device for detecting microorganisms in a sample, comprising:
a lysis section for dissolving microorganisms in the sample; and
a separation section for separating out a nucleic acid from a lysate of microorganisms formed in the lysis section communicating with the lysis section,
wherein the lysis section includes a channel for holding a bacteriolytic agent for lysing microorganisms, and the separation section includes a separation means capable of separating out a nucleic acid contained in the lysate from other substances.

2. The device according to claim 1, wherein the bacteriolytic agent contains a surfactant.

3. The device according to claim 1, wherein the separation means of the separation section is a separation means using a molecular size, a separation means using centrifugation, or a separation means using hydraulic filtration.

4. The device according to claim 1, further comprising:
a complex forming section for forming a protein-nucleic acid complex from the nucleic acid separated in the separation section communicating with the separation section; and
a detection section including a means for detecting the complex communicating with the complex forming section.

5. The device according to claim 4, wherein the complex forming section includes a channel holding a specific nucleic acid derived from the microorganisms and a substance capable of forming a protein-nucleic acid complex.

6. The device according to claim 5, wherein the substance is CRISPR-dCas9 to which sgRNA specific to the specific nucleic acid is bound, or a protein marker that specifically binds to the specific nucleic acid.

7. The device according to claim 6, wherein the detection section includes a means for electrochemically or optically detecting the complex.

8. The device according to claim 6, wherein the detection section detects the complex using a nanopore.

9. The device according to claim 1, further comprising:
a detection section including a means for detecting the nucleic acid separated in the separation section communicating with the separation section.

10. The device according to claim 9, wherein the detection section detects the nucleic acid by real-time PCR or an immunological method.

11. A method for detecting microorganisms in a sample using the device according to any one of claims 1 to 8.

12. The method according to claim 11, comprising:
a lysis step of lysing microorganisms in a sample in the lysis section; and
a separation step of separating out a nucleic acid from a lysate of microorganisms obtained in the lysis section, in the separation section.

13. The method according to claim 12, further comprising:
a complex forming step of forming a protein-nucleic acid complex from the nucleic acid separated in the separation section, in the complex forming section.

14. The method according to claim 13, further comprising:
a detection step of detecting the protein-nucleic acid complex formed in the complex forming section, in the detection section.

15. A method for detecting microorganisms in a sample using the device according to any one of claims 1 to 3, 9, and 10.

16. The method according to claim 15, comprising:
a lysis step of lysing microorganisms in a sample in the lysis section;
a separation step of separating out a nucleic acid from a lysate of microorganisms obtained in the lysis section, in the separation section; and
a detection step of detecting the nucleic acid separated in the separation section, in the detection section.
